# EUROPÄISCHE PATENTSCHRIFT

(11) **EP 2 594 220 B1**
(45) Veröffentlichungstag und Bekanntmachung des Hinweises auf die Patenterteilung: **19.08.2020**
(21) Anmeldenummer: 11189085.1
(22) Anmeldetag: 15.11.2011
(51) Int. Cl.: A61B 1/12

(54) **VERBINDUNGSVORRICHTUNG FÜR EINE REINIGUNGSVORRICHTUNG ZUR REINIGUNG VON INSTRUMENTEN MIT WENIGSTENS EINEM ZU REINIGENDEN LUMEN**
CONNECTION DEVICE FOR A CLEANING DEVICE FOR CLEANING INSTRUMENTS WITH AT LEAST ONE LUMEN TO BE CLEANED
DISPOSITIF DE RACCORDEMENT POUR UN DISPOSITIF DE NETTOYAGE D'INSTRUMENTS DOTÉS D'AU MOINS UNE LUMIÈRE DEVANT ÊTRE NETTOYÉE

(43) Veröffentlichungstag der Anmeldung: 22.05.2013
(73) Patentinhaber: Belimed AG, 6300 Zug (CH)
(72) Erfinder: WOLF, Kurt, 6014 Littau (CH); MUTTENHAMMER, Maximilian, 6003 Luzern (CH)
(74) Vertreter: Hepp Wenger Ryffel AG

(56) Entgegenhaltungen:
- US-A- 5 310 524
- US-A1- 2004 134 520
- US-A1- 2007 100 204

## Beschreibung

Die Erfindung betrifft eine Reinigungsvorrichtung zur Reinigung von Instrumenten mit wenigstens einem zu reinigenden Lumen, insbesondere chirurgischen Instrumenten, vorzugsweise Endoskopen, sowie ein Verfahren zum Reinigen von Instrumenten, gemäss den Oberbegriffen der unabhängigen Ansprüche.

Bei den zur Aufbereitung vorgesehenen Instrumenten kann es sich beispielsweise um humanmedizinische oder veterinärmedizinische Endoskope handeln, im Einzelfall aber auch um andere Instrumente für industrielle oder wissenschaftliche Zwecke, welche Kanäle bzw. Lumen z.B. in Schläuchen aufweisen, die nach einer bestimmten Einsatzzeit des Instruments gereinigt werden müssen. Insbesondere medizinische Endoskopiegeräte dürfen dabei oft aufgrund einer thermischen Instabilität der Endoskopschläuche ohne thermische Sterilisation nur chemisch desinfiziert werden. Unter einem Aufbereitungsprozess wird bei derartigen Endoskopen daher üblicherweise ein Prozess verstanden, der im Wesentlichen eine Abfolge von Spülen, Reinigen, Desinfizieren und Trocknen umfasst. Die spezifischen Anforderungen an und die Prüfverfahren für Endoskop-Reinigungsgeräte mit chemischer Desinfektion sind beispielsweise in der Norm EN ISO 15883, Teil 4 (ISO 15883-4:2009) festgelegt.

Die Effektivität der Reinigung des Lumens kann bei derartigen Instrumenten nicht durch Inspektion unmittelbar beurteilt werden. Um dennoch sicherzustellen, dass in den Kanälen keine Verunreinigungen zurückbleiben, wird in vielen Fällen während des Reinigungsprozesses eine Durchlässigkeit der Kanäle als Mass der Reinheit geprüft. Das Mass beruht auf der Annahme, dass bei ausreichender Durchgängigkeit auch eine ausreichende Reinigung bzw. Desinfektion erfolgt.

Beispielsweise beschreibt EP 2 243 418 A1 eine Reinigungsvorrichtung mit einer integrierten Strömungsmessung in jeder Zuführung zu einem gehäusefesten Versorgungsanschluss für ein Lumen des Instruments. Somit kann die Strömung zu jedem Anschluss einzeln überwacht werden. Ist das (fluiddicht) angeschlossene Lumen ganz oder teilweise blockiert, kommt die Strömung zum Stillstand oder fällt unter einen vorgegebenen Wert ab. Eine Reinigung gilt in diesem Fall als nicht erfolgt bzw. ungenügend, sodass das Instrument nicht freigegeben werden kann. Derartige Vorrichtungen sind allerdings kompliziert in der Anwendung, konstruktiv aufwändig und vergleichsweise teuer.

Ausserdem lässt die auf diese Weise erreichte Überwachung des Reinigungsvorgangs Rückschlüsse auf eine tatsächlich erfolgte Reinigung nur dann zu, wenn eine fluiddichte Verbindung zwischen Strömungsmessungsbereich und Lumen besteht. Oft sind jedoch Verbindungsvorrichtungen zwischen dem Versorgungsanschluss der Reinigungsvorrichtung und dem Lumen angeordnet wie z.B. Schläuche oder Pumpen (siehe unten), über welche ein von einer Reinigungsvorrichtung bereitgestelltes flüssiges Medium wie z.B. ein Reinigungs- und/oder Desinfektionsmittel in das Lumen des Instruments geleitet wird. Es besteht somit zwischen einer derartigen Strömungsmessung und Lumen eine Vielzahl von Möglichkeiten für eine Leckage, womit gegebenenfalls trotz blockiertem Lumen in der Zuführung zum Anschluss eine ausreichende Strömung gemessen würde.

Es bedarf daher auch bei derartig aufwändigen Maschinen zur Sicherstellung einer ausreichenden Reinigung eine erhöhte Aufmerksamkeit eines für die Reinigung verantwortlichen Benutzers. Aufgrund der maschinell umfassenden Überwachung besteht allerdings ein gewisses Risiko, dass die erforderliche Aufmerksamkeit eben gerade aufgrund der vermeintlich umfassenden maschinellen Überwachung nachlässt.

US 2004/0134520, auf der der Oberbegriff des Anspruchs 1 basiert, zeigt eine Methode zur Wideraufbereitung von Vorrichtungen mit einem Lumen. Fluid wird über Kanäle und Ausgangsanschlüsse mit verschiedenen Drücken bereitgestellt. Eine Überwachung eines Fluidparameters im Innenraum einer Verteilervorrichtung wird mittels eines im Innenraum der Verteilervorrichtung angeordneten Sensors ermöglicht.
US 5,310,524 zeigt ein System zur Wiederaufbereitung von Katheter. Katheter werden selektiv an einen Quelle für Sterilisationsmittel angeschlossen.
US 2007/0100204 zeigt eine Vorrichtung um die Konnektivität eines Kanals in einem Endoskop zu detektieren, während das Endoskop Wideraufbereitet wird. Dazu wird ein Fluid über einen Anschluss in den Kanal des Endoskopes gebracht und der Druck gemessen.
EP 2 283 789 A1 beschreibt eine Verbindungsvorrichtung, welche zwischen einen Versorgungsanschluss einer Reinigungsvorrichtung und das zu reinigende Lumen geschaltet ist. Die Verbindungsvorrichtung misst über einen internen Drucksensor das Vorhandensein einer Wasserversorgung zwischen Versorgungsanschluss der Reinigungsvorrichtung und angeschlossenem Lumen. Die Verbindungsvorrichtung umfasst eine vom Drucksensor geregelte interne Pumpe, welche bei ausreichendem Wasserdruck in der Verbindungsvorrichtung des Druck im Flüssigkeitsfluss zum Lumen erhöht.

Eine derartige Verbindungsvorrichtung ist aufwändig in der Konstruktion und vergleichsweise teuer und aufgrund der Komplexität auch anfällig für mechanische Beschädigung. Ausserdem sind zu ihrem Betrieb externe Anschlüsse wie beispielsweise eine Stromversorgung und gegebenenfalls Steuerungsanschlüsse erforderlich.

Die Aufgabe der Erfindung ist es daher, die genannten Nachteile des Standes der Technik zu überwinden und insbesondere eine kostengünstige Vorrichtung bereitzustellen, welche auf einfache Weise eine ausreichende Überwachung der Reinigung und Desinfektion von Instrumenten mit einem Lumen, insbesondere im medizinischen Bereich, ermöglicht. Dabei sollen insbesondere die Vorgaben der relevanten Normen erfüllt werden können.

Diese Aufgaben der Erfindung werden durch eine Vorrichtung mit den Merkmalen des Anspruchs 1 gelöst. Dieser betrifft eine Reinigungsvorrichtung zur Reinigung von Instrumenten mit wenigstens einem zu reinigenden Lumen, insbesondere chirurgischen Instrumenten, vorzugsweise Endoskopen. Die Reinigungsvorrichtung umfasst wenigstes einen Versorgungsanschluss, über welchen ein flüssiges Medium bereitstellbar ist, und welcher zum Anschluss einer Verbindungsvorrichtung ausgebildet ist. Die Verbindungsvorrichtung weist einen Innenraum mit einem Eingangsanschluss und wenigstens einem Ausgangsanschluss auf, wobei der Eingangsanschluss direkt oder indirekt an einen Versorgungsanschluss der Reinigungsvorrichtung anschliessbar ist, über welchen ein flüssiges Medium bereitgestellt wird. Der Ausgangsanschluss ist direkt oder indirekt an das zu reinigende Lumen des Instruments anschliessbar. Die Verbindungsvorrichtung umfasst eine Einrichtung, welche zur Überwachung wenigstens eines Fluidparameters des flüssigen Mediums im Innenraum eine direkte oder indirekte Wechselwirkung des flüssigen Mediums mit einem externen Überwachungssensor der Reinigungsvorrichtung ermöglicht.

Die erfindungsgemässe Verbindungsvorrichtung erlaubt die Überwachung eines Fluidparameters wie beispielsweise eines Drucks oder eines Flusses im Innenraum der Verbindungsvorrichtung. Damit kann überwacht bzw. sichergestellt werden, dass der Eingangsanschluss der Verbindungsvorrichtung an den Versorgungsanschluss der Reinigungsvorrichtung korrekt angeschlossen ist und z.B. ausreichend flüssiges Medium oder flüssiges Medium mit genügend Druck zu dem Ausgangsanschluss der Verbindungsvorrichtung, d.h. zum angeschlossenen Lumen gefördert wird. Selbstverständlich können dabei mehrere Ausgangsanschlüsse für mehrere Lumen an der Verbindungsvorrichtung vorgesehen sein.

Der Erfindung liegt der Gedanke zugrunde, eine als separates Teil ausführbare und beispielsweise aus dem Reinigungsraum entnehmbare Verbindungsvorrichtung als passives Element d.h. ohne elektrische Komponenten oder interne Signalverarbeitung möglichst kostengünstig und ohne sensible Bauteile mechanisch robust auszubilden. Der Überwachungssensor sowie zugehörige Auswertungselektronik etc. können fest in der stationären Reinigungsvorrichtung angeordnet sein. Insbesondere kann der Überwachungssensor unabhängig von der Verbindungsvorrichtung und ohne im Reinigungsraum verlaufende Versorgungs- und/oder Informationsleitungen, beispielsweise in einer Seitenwand des Reinigungsraums angeordnet, fest verbaut sein.

Die erfindungsgemässe Einrichtung der Verbindungsvorrichtung bildet dabei eine Schnittstelle zum Überwachungssensor, welche konstruktiv einfach ausgestaltet sein kann. Beispielsweise kann die Einrichtung einen Druckindikator umfassen, welcher bei Vorhandensein eines Minimaldrucks z.B. mechanisch auf den Überwachungssensor wirkt. Ebenso ist es denkbar, einen gerichtet Strahl des flüssigen Mediums direkt auf den z.B. auf Druck empfindlichen Überwachungssensor zu leiten. Erfindungsgemäss kann die Verbindungsvorrichtung somit als auswechselbarer Gebrauchsgegenstand besonders kostengünstig hergestellt werden.

Aufgrund der Einrichtung der erfindungsgemässen Verbindungsvorrichtung kann diese bei fest im Reinigungsraum angeordnetem Überwachungssensor aus dem Reinigungsraum entnommen werden. Eine Vorbereitung, z.B. ein Anschluss des Lumens, kann ausserhalb der Reinigungsmaschine erfolgen. Damit kann ein mit der Reinigung betrauter Benutzer bei der Vorbereitung in einer angenehmen Körperhaltung entspannt und damit auch sorgfältiger arbeiten, als es beispielsweise bei fest im Reinigungsraum angeordneten und oft umständlich zugänglichen Ausgangsanschlüssen der Fall ist. Insbesondere ist es für den Benutzer damit einfacher, eine Qualität der Anschlüsse zu prüfen und sicherzustellen, dass das Lumen korrekt angeschlossen ist.

Vorzugsweise erlaubt die Einrichtung dabei eine Überwachung eines vorgegebenen Minimalwerts des wenigstens einen Fluidparameters. Insbesondere kann die Überwachung im Sinne einer binären Ja/Nein-Messung des Vorhandenseins eines Wertes oberhalb oder unterhalb des Minimalwerts erfolgen. Damit kann sichergestellt werden, dass beispielsweise eine Pumpe der Reinigungsvorrichtung das flüssige Medium wie vorgesehen in die Verbindungsvorrichtung fördert oder ob ein ausreichender Druck für eine effiziente Reinigung an den Ausgangsanschlüssen anliegt. Insbesondere kann auch überwacht werden, ob die Verbindungsvorrichtung korrekt an den Versorgungsanschluss der Reinigungsvorrichtung angeschlossen ist.

Bevorzugt ist der wenigstens eine überwachte Fluidparameter ein Druck, insbesondere umfassend einen dynamischen Druck. Im Folgenden ist die Erfindung ohne Beschränkung der Allgemeinheit anhand einer Drücküberwachung beschrieben, wobei grundsätzlich wie erwähnt auch andere Fluidparameter überwacht werden können. Bevorzugt arbeitet die Einrichtung der Verbindungsvorrichtung derart bzw. ist derart einstellbar, dass unabhängig davon, ob am Ausgangsanschluss ein Lumen angeschlossen ist, über die Einrichtung ein ausreichender Druck signalisiert werden kann. Dies hat den Vorteil, dass bei mehreren Ausgangsanschlüssen allenfalls unbenutzte Anschlüsse, d.h. Anschlüsse, an welche kein Lumen angeschlossen ist, nicht gesondert verschlossen werden müssen. Da keine Überwachung das eigentlichen Durchgangs des flüssigen Mediums durch das Lumen erfolgt, muss ein mit der Reinigung betrauter Benutzer bei der Vorbereitung der Reinigung sicherstellen, dass das Lumen vor der Reinigung einen vorgegebenen Zustand aufweist, d.h. insbesondere dass das Lumen ausreichend durchgängig ist und fluiddicht am Ausgangsanschluss angeschlossen ist.

Erfindungsgemäss ist die Einrichtung der Verbindungsvorrichtung dabei derart ausgebildet, dass die Überwachung des Fluidparameters ohne eine beständige Verbindung zwischen Einrichtung und externem Überwachungssensor erfolgen kann. Mit "beständig" ist hier eine Verbindung bezeichnet, welche vor der Durchführung des Reinigungsvorgangs hergestellt wird und bis nach der Durchführung des Reinigungsvorgangs bestehen bleibt. Die Einrichtung der erfindungsgemässen Verbindungsvorrichtung ist hingegen bevorzugt derart ausgebildet, dass sie die oben genannte direkte oder indirekte Wechselwirkung nur bei Überschreiten und/oder Unterschreiten eines Minimalwerts des überwachten Fluidparameters ermöglicht.

Dies hat den Vorteil, dass die Verbindungsvorrichtung vom Benutzer vor der Reinigung nur an den Versorgungsanschluss angeschlossen werden muss, ohne dass eine zusätzliche Verbindung mit dem Überwachungssensor hergestellt werden muss. Insbesondere muss damit bei der Entnahme der Verbindungsvorrichtung aus einer Reinigungsvorrichtung auch keine derartige Verbindung gelöst werden.

Bevorzugt ist die Einrichtung der Verbindungsvorrichtung derart ausgebildet, dass die direkte oder indirekte Wechselwirkung eine mechanische Wirkverbindung ist, insbesondere eine Druckkraft.

Aufgrund der Druckkraft kann der externe Überwachungssensor als Drucksensor ausgebildet sein, welche vielfältig und an die Anwendung in einem nassen Umfeld angepasst auf dem Markt erhältlich sind. Eine mechanische Wirkverbindung zur Übertragung einer Druckkraft ist zudem einfach herzustellen (siehe unten, Betätigungsstift oder Wasserstrahl).

In anderen Varianten kann je nach Erfordernis z.B. auch eine optische Überwachung eines Fluidparameters zur Anwendung kommen, in welchem Fall die Einrichtung z.B. ein Sichtfenster umfasst, welches z.B. die Sicht auf ein im Innenraum angeordnetes Flügelrad freigibt. Ebenso ist eine berührungslose Überwachung mittels Ultraschall oder Magnetfelder denkbar. Da derartige Sensoren und/oder Einrichtungen allerdings vergleichsweise aufwendig und damit auch kostenintensiv sind, ist in der Regel eine Überwachung mittels mechanischer Wirkverbindung bevorzugt.

Wie bereits erwähnt, kann die Verbindungsvorrichtung mit Vorteil auch mehrere zum Anschluss mehrerer zu reinigender Lumen eines oder mehrerer Instrumente vorhanden sind. Dies ist insbesondere bei Instrumenten mit mehreren Lumen vorteilhaft, da diese gemeinsam gereinigt werden können.
Bevorzugt umfasst die Verbindungsvorrichtung wenigstens einen länglichen Rohrabschnitt, in welchem der Innenraum ausgebildet ist und an dessen einen Längsende, insbesondere stirnseitig, die Einrichtung angeordnet ist. Für Fluidanwendungen sind Rohrabschnitte, insbesondere mit kreiszylindrischen Querschnitten, besonders vorteilhaft, da sie einfach zu verarbeiten, strukturell stabil sind und eine optimale Platzausnutzung ermöglichen.

Vorzugsweise ist dabei der Ausgangsanschluss oder, im Falle mehrerer Ausgangsanschlüsse, sind die Ausgangsanschlüsse auf einer Mantellinie angeordnet. Im Falle mehrerer Ausgangsanschlüsse sind diese bevorzugt längs der Mantellinie in Längsrichtung gleichmässig verteilt. Zum einfachen Anschluss weisen die Ausgangsanschlüsse mit Vorteil jeweils einen bezüglich der Längsachse des wenigstens einen Rohrabschnitts weitgehend radial abstehenden Anschlussstutzen auf. Die Anschlussstutzen können dabei kraft-, form- oder stoffschlüssig mit dem Rohrabschnitt fest verbunden sein wie z.B. verschweisst oder genietet, eingeschraubt, eingesteckt oder nach Art eines Bajonetts verriegelt.

Vorzugsweise erfolgt der Anschluss an den Anschlussstutzen über ein Kopplungselement, welches zunächst mit dem Lumen und in der Folge mit dem Anschlussstutzen verbunden wird. Dies hat den Vorteil, dass die heikle Verbindung zwischen Lumen und Kopplungsteil ohne Einschränkung von z.B. Lage oder Ausrichtung etc. erstellt werden kann. Die Verbindung zum Anschlussstutzen, welche hingegen gewisse Vorgaben hinsichtlich Anordnung und Ausrichtung hat, kann über eine standardisierte Kupplung erfolgen.

Es wird daher ein Kopplungselement zum Verbinden eines Lumens mit einem Anschlussstutzen vorgeschlagen, bei welchem ein am Kopplungselement gehaltertes ringförmiges elastisches Element eine Doppelfunktion zur gleichzeitigen Arretierung und Dichtung der Verbindung erfüllt, wobei diese Doppelfunktion durch einen Eingriff des elastischen Elements in eine entsprechende Nut des Anschlussstutzens erfolgt. Insbesondere wird durch den Eingriff des elastischen Elements in die Nut auch eine haptische Rückmeldung an den Benutzer gegeben, wenn die Verbindung zwischen Kopplungsteil und Anschlussstutzen hergestellt ist. Bevorzugt ist das ringförmige elastische Element in einem Aufnahmeraum des Kopplungselements für einen Kopplungsbereich des Anschlussstutzens in einer innen umlaufenden Nut gehaltert, wobei ein Innendurchmesser der ringförmigen Öffnung des elastischen Elements kleiner ist, als ein Innendurchmesser des Aufnahmeraums.

Es versteht sich, dass zur Verbindung des Lumens mit dem Anschlussstutzen z.B. ein das Lumen aufweisender Schlauch direkt auf einen entsprechend ausgebildeten Kopplungsbereich des Anschlussstutzens aufgesteckt werden kann.

Im Falle wenigstens eines länglichen Rohrabschnitts des Gehäuses kann das Gehäuse mit Vorteil einen weiteren länglichen Rohrabschnitt umfassen, welcher mit einem seiner Längsenden weitgehend rechtwinklig an den wenigstens einen Rohrabschnitt anschliesst. Am freien Ende des weiteren Rohrabschnitts ist bevorzugt der Eingangsanschluss ausgebildet, wobei das Gehäuse vorzugsweise als L-förmiges Rohrstück, vorteilhaft mit kreiszylindrischem Querschnitt, ausgebildet ist.

Ein L-förmiges Gehäuse hat den Vorteil, dass es mit einem der freien Enden z.B. bei einer Innenwand eines weitgehend quaderförmigen Reinigungsraums angeordnet sein kann und mit dem anderen freien Ende bei einer benachbarten Innenwand, während beide Rohrabschnitte weitgehend senkrecht zur entsprechenden Innenwand ausgerichtet sind. Damit lässt sich die Verbindungsvorrichtung z.B. auf einfache Weise von vorne her an einen an der Rückwand angeordneten Versorgungsanschluss anschliessen (z.B. einstecken), während das Längsende mit der Einrichtung auf eine Seitenwand ausgerichtet ist, an welcher der externe Überwachungssensor angebracht ist. Es versteht sich, dass z.B. auch eine T-Form denkbar ist, bei welcher der weitere Rohrabschnitt mit einem Längsende z.B. in Längsrichtung des wenigstens einen Rohrabschnitts mittig an diesen anschliesst. Ebenso ist es denkbar, dass das Gehäuse U-förmig ausgebildet ist, sodass beide freien Enden der U-Form bei derselben Innenwand des Reinigungsraums angeordnet sein können, sodass z.B. Versorgungsanschluss und Überwachungssensor auf derselben Innenwand ausgebildet sein können. Bevorzugt kommen mit einer Reinigungsvorrichtung mehrere Verbindungsvorrichtungen zum Einsatz. So kann z.B. während der Reinigung eines Instruments in der Reinigungsvorrichtung bereits ein weiteres ausserhalb der Reinigungsvorrichtung vorbereitet werden. Es ist daher von Vorteil, wenn die Verbindungsvorrichtung möglichst kostengünstig ausgebildet ist. Bei einer besonders einfachen und kostengünstigen Ausführung der Einrichtung der Verbindungsvorrichtung umfasst diese einen gegen eine Rückstellkraft, insbesondere eine Federkraft, verschiebbar im Innenraum gelagerten Stössel, welcher aufgrund des wenigstens einen Fluidparameters, insbesondere eines Drucks, unterschiedliche Verschiebestellung einnehmen kann. Aufgrund der unterschiedlichen Verschiebestellungen wird die Wechselwirkung mit dem externen Überwachungssensor ermöglicht.

In den unterschiedlichen Verschiebestellungen ragt der Stössel mit einem Betätigungsstift unterschiedlich weit aus der Verbindungsvorrichtung hervor. Je nach Abstand zu einem entsprechend angeordneten externen Überwachungssensor, kann somit in einer bestimmten Verschiebestellung über den Stössel eine mechanische Wirkverbindung mit dem Überwachungssensor hergestellt werden. Wird zudem eine ausreichend grosse Kraft vom flüssigen Medium auf den Stössel ausgeübt, kann über den Betätigungsstift der beispielsweise als Druckschalter ausgebildete Überwachungssensor betätigt werden. Der Überwachungssensor kann somit einer Steuerungsvorrichtung das Vorhandensein eines ausreichenden Drucks signalisieren. Der Stössel kann auf einfache Weise einen im Innenraum angeordneten Kolben umfassen, welcher z.B. den Druck des flüssigen Mediums aufnimmt, wobei der Betätigungsstift fest mit dem Kolben verbunden ist. Der Betätigungsstift kann dabei gleichzeitig eine Funktion als Führungselement für eine die Rückstellkraft bereitstellende Feder erfüllt.

Besonders bevorzugt ist im Falle wenigstens eines länglichen Rohrabschnitts der Stössel in Längsrichtung des wenigstens einen Rohrabschnitts verschiebbar und der Betätigungsstift ragt in Längsrichtung über das Längsende des wenigstens einen Rohrabschnitts hinaus. Auf diese Weise kann der Innenraum der Verbindungsvorrichtung selbst eine Verschiebeführung für den Kolben des Stössels bereitstellen, wobei der fest mit dem Kolben verbundener Betätigungsstift durch eine Öffnung in einem Endabschluss des Rohrabschnitts je nach Verschiebestellung unterschiedlich weit nach aussen ragt.

Im Falle einer Überwachung des Drucks ist eine Druckdifferenz zwischen einem Bereich in Verschieberichtung vor und hinter dem Kolben erforderlich. Hierzu können beispielsweise der Endabschluss des Rohrabschnitts, durch welchen der Betätigungsstift austritt, oder der wenigstens eine Rohrabschnitt selbst eine Druckentlastungsöffnung aufweisen.

In einer anderen, je nach Anwendung ebenfalls bevorzugten Ausführung, umfasst die Einrichtung eine Düsenöffnung, durch welche das im Innenraum vorhandene flüssige Medium als gerichteter Strahl aus dem Innenraum austreten kann. Bevorzugt ist dabei im Falle des wenigstens einen Rohrabschnitts die Düsenöffnung derart ausgebildet, dass der gerichtete Strahl an dem Längsende des wenigstens einen Rohrabschnitts, an welchem die Einrichtung angeordnet ist, im Wesentlichen in dessen Längsrichtung austritt, um die Wechselwirkung mit dem externen Überwachungssensor zu ermöglichen.

Eine derartige Ausführung hat den Vorteil, dass die Einrichtung der Verbindungsvorrichtung keine mechanisch beweglichen Teile umfasst. Das aus dem Innenraum durch die Düsenöffnung austretende flüssige Medium kann bei ausreichend grossem Druck beim Aufprall auf einen beabstandet angeordneten Drucksensor oder Druckschalter eine messbare Kraft ausüben bzw. eine ausreichend grosse Kraft, um den Druckschalter zu betätigen und somit als Signalmittel wirken.

Mit besonderem Vorteil ist die Verbindungsvorrichtung direkt in einem Reinigungskorb angeordnet, welcher in den Reinigungsraum eingebracht werden kann. Die Erfindung betrifft daher auch einen Reinigungskorb zum Einbringen in einen Reinigungsraum einer Reinigungsvorrichtung, mit einem Aufnahmebereich für ein Instrument mit einem zu reinigenden Lumen, insbesondere ein chirurgisches Instrument, vorzugsweise ein Endoskop, wobei der Reinigungskorb eine vorliegend beschriebene Verbindungsvorrichtung umfasst, welche insbesondere fest mit dem Reinigungskorb verbunden ist.

Dies hat den Vorteil, dass das Instrument im Aufnahmebereich angeordnet werden kann und alle erforderlichen Verbindungen der Verbindungsvorrichtung mit dem oder den Lumen des Instruments, ausserhalb der Reinigungsvorrichtung z.B. auf einem Arbeitstisch hergestellt werden können. Es versteht sich, dass der Reinigungskorb sowie die Verbindungsvorrichtung derart ausgebildet sein können, dass mehrer Instrumente in einem Aufnahmebereich angeordnet und angeschlossen werden können. Das derart vorbereitete Instrument im Reinigungskorb braucht vor einer Reinigung nicht mehr manipuliert zu werden - alle weiteren Fluidanschlüsse erfolgen über die Verbindungsvorrichtung, bevorzugt automatisch, beim Einbringen des Reinigungskorbs in den Reinigungsraum.

Mit Vorteil ist die Verbindungsvorrichtung hierzu mit der Einrichtung und dem Eingangsanschluss jeweils in einem Randbereich des Aufnahmebereichs des Reinigungskorbs angeordnet, sodass die Einrichtung und gegebenenfalls der Eingangsanschluss von ausserhalb des Aufnähmebereichs her zugänglich sind. Randbereich bezeichnet hierbei beispielsweise einen Bereich bei einem Rand einer Grundfläche des Aufnahmebereichs, z.B. bei einer den Aufnahmebereich begrenzenden Umrandung.

Bevorzugt ist der Reinigungskorb dabei schubladenartig mit einer weitgehend rechteckigen Grundfläche ausgebildet und weist eine Umrandung mit mehreren Wandabschnitten auf, sodass der Reinigungskorb in einer entsprechend ausgebildeten Aufnahme eines Einsatzwagens oder des Reinigungsraums nach Art einer Schublade ein- und ausschiebbar ist. Einsatzwagen bezeichnet hierbei einen in den Reinigungsraum einbringbaren Wagen oder ein derartiges Gestell, in welchen die Reinigungskörbe eingesetzt werden können. Der Einsatzwagen kann dabei z.B. vollständig aus dem Reinigungsraum ausgebracht werden oder auch nur ausfahrbar bzw. ausziehbar im Reinigungsraum gelagert sein.

Bevorzugt umfassen die Wandabschnitte und/oder die Grundfläche ein vergleichsweise feinmaschiges Raster wie beispielsweise ein siebartiges Gitter. Das Gitter hat den Vorteil, dass sich kein flüssiges Medium im Reinigungskorb sammelt und abfliessen kann. Ausserdem kann das Gitter gleichzeitig auch als Raster für Halterungselemente dienen, welche beispielsweise modular angeordnet werden können und das Instrument während der Reinigung haltern. "Feinmaschig" bezeichnet hier eine Maschengrösse, welche im Vergleich zu den Dimensionen der Elemente des Instruments im Wesentlichen klein ist, sodass das Instrument sicher im Aufnahmebereich unterstützt ist.
Mit Vorteil ist die Verbindungseinrichtung mit dem Eingangsanschluss bei einem hinteren Wandabschnitt und mit der Einrichtung an einem seitlichen Wandabschnitt angeordnet, wobei bei der Einrichtung ein Durchbruch im seitlichen Wandabschnitt und bei dem Eingangsanschluss ein Durchbruch im hinteren Wandabschnitt ausgebildet ist. Auf diese Weise sind Einrichtung sowie Eingangsanschluss durch die Durchbrüche von aussen her zugänglich. Die Durchbrüche können dabei derart ausgebildet sein, dass z.B. der Versorgungsanschluss der Reinigungsvorrichtung im Durchbruch aufgenommen sein kann, wenn der Eingangsanschluss an diesen angeschlossen ist. Bevorzugt sind die Einrichtung sowie der Eingangsanschluss wenigstens teilweise im jeweiligen Durchbruch angeordnet.

Ein "hinterer" Wandabschnitt bezeichnet hierbei einen Wandabschnitt, welcher in Einbringrichtung vorne am Reinigungskorb angeordnet ist und bei in betriebsbereiter Anordnung des Reinigungskorbs in der Reinigungsvorrichtung bei einer Rückwand angeordnet ist. Entsprechend bezeichnet ein "seitlicher" Wandabschnitt einen bezüglich der Einbringrichtung seitlich angeordneten Wandabschnitt, welcher in betriebsbereiter Anordnung des Reinigungskorbs in der Reinigungsvorrichtung bei einer Seitenwand angeordnet ist.

Mit besonderem Vorteil umfasst der Reinigungskorb eine Anschlussvorrichtung mit einem Eingangsanschluss vorhanden ist, an welchen direkt oder indirekt eine Dichtigkeitsmessvorrichtung der Reinigungsvorrichtung anschliessbar ist, und mit einem Ausgangsanschluss, an welchen direkt oder indirekt eine auf Dichtigkeit zu überprüfende Kavität des Instruments anschliessbar ist.

Insbesondere im medizinischen Bereich umfassen Instrumente häufig eine äussere Umhüllende, welche beispielsweise mehrer Schläuche fluiddicht umhüllt. Bei der Aufbereitung derartiger Instrumente wird die Umhüllende in der Regel auf ihre Dichtigkeit geprüft. Aufgrund der Anschlussvorrichtung kann bei der Reinigungsvorbereitung somit ein Anschluss zwischen der Kavität des im Aufnahmebereich angeordneten Instruments mit der Anschlussvorrichtung hergestellt werden. Wird der Reinigungskorb mit an den Ausgangsanschluss der Anschlussvorrichtung angeschlossener Kavität des Instruments in die Reinigungsraum eingebracht, muss nur noch der Eingangsanschluss der Anschlussvorrichtung mit der Dichtigkeitsmessvorrichtung der Reinigungsvorrichtung verbunden werden - eine Manipulation des Instruments ist dabei nicht mehr erforderlich. Zusammen mit der erfindungsgemässen Verbindungsvorrichtung kann das Instrument bei der Reinigungsvorbereitung somit derart präpariert werden, dass beim Einbringen in den Reinigungsraum und zur Durchführung des Reinigungsvorgangs keine weitere Manipulation des Instruments mehr erforderlich ist.

Vorzugsweise ist die Anschlussvorrichtung an einem der Wandabschnitte derart befestigt, dass der Ausgangsanschluss dem Aufnahmebereich zugewandt ist und der Eingangsanschluss im Wesentlichen nach aussen gerichtet ist. Bevorzugt ist die Anschlussvorrichtung an einem vorderen Wandabschnitt angeordnet, d.h. einem Wandabschnitt, welcher dem hinteren Wandabschnitt gegenüberliegt und bei betriebsbereiter Anordnung des Reinigungskorbs im Reinigungsraum von einer Zugangsöffnung des Reinigungsraums her einfach zugänglich ist.

Bevorzugt umfasst der Reinigungskorb ein Signalmittel zur Signalisierung einer Präsenz des Reinigungskorbs zur Detektierung durch einen entsprechenden Präsenzdetektor der Reinigungsvorrichtung, insbesondere ein passives magnetisches Signalmittel. In Varianten kann das Signalmittel auch zusätzlich zur Präsenz des Reinigungskorbes weitere Information signalisieren und kann hierzu beispielsweise auf der bekannten RFID-Technologie (kurz für "radio-frequency identification") basierende Elemente umfassen. Ebenso sind andere Signalisierungsmittel wie z.B. optisch lesbare Strichcodes denkbar.

Das Signalmittel ermöglicht der Reinigungsvorrichtung festzustellen, ob in einer dafür vorgesehenen Aufnahme ein Reinigungskorb angeordnet ist. Eine negative Rückmeldung des externen Überwachungssensors zur Überwachung des Fluidparameters kann damit von einer Steuerungsvorrichtung der Reinigungsvorrichtung mit der Präsenz eines Reinigungskorbs abgeglichen werden.
Die Erfindung betrifft eine Reinigungsvorrichtung zur Anwendung in Verbindung mit der Verbindungsvorrichtung bzw. einem mit der Verbindungsvorrichtung versehenen Reinigungskorb. Dieser Aspekt der Erfindung betrifft eine Reinigungsvorrichtung mit einem Reinigungsraum zur Reinigung von Instrumenten mit wenigstens einem zu reinigenden Lumen, insbesondere chirurgischen Instrumenten, vorzugsweise Endoskopen. Dabei ist wenigstens ein Versorgungsanschluss vorhanden, über welchen ein flüssiges Medium bereitstellbar ist, und welcher zum direkten oder indirekten Anschluss einer Verbindungsvorrichtung wie vorliegend beschrieben ausgebildet ist. Im Reinigungsraum ist wenigstens ein Überwachungssensor derart angeordnet, dass zur Überwachung wenigstens eines Fluidparameters eines flüssigen Mediums im Innenraum der Verbindungsvorrichtung über deren Einrichtung eine direkte oder indirekte Wechselwirkung des flüssigen Mediums mit dem Überwachungssensor der Reinigungsvorrichtung herstellbar ist, wenn die Verbindungsvorrichtung in betriebsbereitem Zustand direkt oder indirekt an den wenigstens einen Versorgungsanschluss der Reinigungsvorrichtung angeschlossen ist. Insbesondere ist der wenigstens eine Fluidparameter ein Druck im flüssigen Medium.

Eine derartige Reinigungsvorrichtung bildet das die erfindungsgemässe Verbindungsvorrichtung ergänzende Gegenstück. Die Vorteile der Reinigungsvorrichtung ergeben sich unmittelbar aus den im Zusammenhang mit der erfindungsgemässen Verbindungsvorrichtung und/oder dem Reinigungskorb beschriebenen Merkmalen und Vorteilen.
Mit besonderem Vorteil sind der Versorgungsanschluss der Reinigungsvorrichtung an einer Rückwand des Reinigungsraums und der Überwachungssensor an einer Seitenwand des Reinigungsraums angeordnet.

Indem der Versorgungsanschluss an der Rückwand ausgebildet ist, kann eine Anschlussrichtung des Eingangsanschlusses der Verbindungsvorrichtung einer Einschubrichtung der Reinigungskörbe bzw. des Einsatzwagens in eine Aufnahme bzw. den Reinigungsraum entsprechen. Der Eingangsanschluss der Verteilungsvorrichtung bzw. der Verteileranschluss des Einsatzwagens kann somit als zur Rückwand hin ausgerichteter Anschlussstutzen ausgebildet sein, welcher beim ordnungsgemässen Einbringen in den Reinigungsraum in eine Öffnung des Versorgungsanschlusses eingebracht wird.Wie bereits erwähnt, umfasst der Überwachungssensor der Reinigungsvorrichtung bevorzugt einen Druckschalter, welcher durch eine Druckkraft betätigbar ist. In diesem Fall ist die Einrichtung der zur Anwendung kommenden Verbindungsvorrichtung bevorzugt derart ausgebildet, dass eine Druckkraft auf den Überwachungssensor ausübbar ist. Der Überwachungssensor sowie die Einrichtung können somit besonders einfach ausgebildet sein und z.B. über den oben genannten Betätigungsstift oder über einen Flüssigkeitsstrahl eine Druckkraft auf den Überwachungssensor ausüben. Bei Ausführung mit z.B. optischer oder magnetischer Überwachung sind die Überwachungssensoren entsprechend der Überwachungsweise ausgebildet.
Bevorzugt umfasst die Reinigungsvorrichtung eine Steuerungseinheit zur Steuerung der Reinigungsvorrichtung vorhanden ist, welche derart ausgebildet und mit dem Überwachungssensor verbunden ist, dass aufgrund eines Signals des Überwachungssensors durch die Steuerung feststellbar ist, ob ein zur Durchführung eines Reinigungsvorgangs vorgegebener Minimalwert des wenigstens einen Fluidparameters des flüssigen Mediums im Innenraum der Verbindungsvorrichtung überschritten ist. Die Steuerungsvorrichtung kann gemäss den Steuerungsvorgaben in Abhängigkeit der Sensorsignale z.B. bei einem zu geringen Druck eine ungenügende Reinigung protokollieren und/oder einen Alarm für einen Benutzer auslösen und/oder eine Zusatzpumpe einschalten. Es erschliesst sich dabei offensichtlich, dass auch weitere Massnahmen von der Steuerungsvorrichtung aufgrund eines entsprechenden Signals des Überwachungssensors veranlasst werden können.
Bevorzugt weist die Reinigungsvorrichtung im Reinigungsraum eine Aufnahme zur Anordnung von einem vorliegend beschriebenen Reinigungskorb auf. In betriebsbereiter Anordnung des Reinigungskorbs in der Aufnahme ist die Verbindungsvorrichtung betriebsbereit direkt oder indirekt an den Versorgungsanschluss angeschlossen. Der Aufnahme ist dabei der Überwachungssensor zugeordnet, derart, dass die Verbindungsvorrichtung mit der Einrichtung bei einem Messbereich des Überwachungssensors angeordnet ist, wenn der Reinigungskorb betriebsbereit in der Aufnahme angeordnet ist und damit auch die Verbindungsvorrichtung betriebsbereit angeschlossen ist. Die Aufnahme kann dabei z.B. Schienen aufweisen, z.B. an den Seitenwänden des Reinigungsraums oder an einem Einsatzwagen, auf welchen die Reinigungskörbe gleitend ein- oder ausgeschoben werden können.

Mit Vorteil sind weitere Aufnahmen vorhanden, welchen analog der ersten Aufnahme jeweils ein Überwachungssensor zugeordnet ist. In diesem Fall ist jeder Aufnahme ein Anschluss zum Bereitstellen eines flüssigen Mediums zugeordnet ist, an welchen die jeweils zugehörige Verbindungsvorrichtung in betriebsbereiter Anordnung des Reinigungskorbs in der jeweiligen Aufnahme betriebsbereit angeschlossen ist.

Jeder zugeordnete Überwachungssensor ist somit derart bezüglich der zugehörigen Aufnahme angeordnet, dass in betriebsbereiter Anordnung eines Reinigungskorbs in dieser Aufnahme die Einrichtung der zugehörigen Verbindungsvorrichtung derart bezüglich des zugeordneten Überwachungssensors angeordnet ist, dass über die Einrichtung eine direkte oder indirekte Wechselwirkung des flüssigen Mediums mit dem jeweils zugeordneten Überwachungssensor der Reinigungsvorrichtung herstellbar ist

Die zugeordneten Anschlüsse sind derart angeordnet, dass in betriebsbereiter Anordnung eines Reinigungskorbs in dieser Aufnahme die zugehörige Verbindungsanordnung betriebsbereit an den Anschluss angeschlossen ist. Die Anschlüsse stellen dabei das flüssige Medium bereit. Die Anschlüsse können direkt in der Reinigungsmaschine ausgebildet sein, d.h. jeweils einen Versorgungsanschluss der Reinigungsvorrichtung bilden, oder können z.B. mit einem zentralen Versorgungsanschluss verbunden sein und von diesem mit flüssigem Medium versorgt werden.

Je nach Dimensionierung der Reinigungsvorrichtung kann eine weitgehend beliebige Anzahl von Aufnahmen vorgesehen sein. Typische Ausführungen umfassen bevorzugt drei Aufnahmen, welche gestapelt angeordnet sind.
Die Aufnahmen müssen nicht direkt im Reinigungsraum ausgebildet sein. Mit Vorteil kann die Reinigungsvorrichtung daher einen Einsatzwagen zur Anordnung im Reinigungsraum umfassen, an welchem die mehreren Aufnahmen sowie die zugeordneten Anschlüsse ausgebildet sind, wobei der Einsatzwagen einen Verteileranschluss aufweist, welcher an den Versorgungsanschluss der Reinigungsvorrichtung anschliessbar ist und über einen Fluidkanal im Einsatzwagen fluidführend mit den zugeordneten Anschlüssen verbunden ist.

Ein Einsatzwagen hat den Vorteil, dass er aus dem Reinigungsraum entnommen und mit den mit Instrumenten versehenen Reinigungskörben bestückt werden kann. Zur Durchführung des Reinigungsvorgangs muss nur der bestückte Einsatzwagen in den Reinigungsraum eingebracht werden, wobei bevorzugt der Verteileranschluss automatisch an den Versorgungsanschluss anschliesst und die gewünschte Positionierung der Einrichtungen Verbindungsvorrichtungen der Reinigungskörbe bei den Überwachungssensoren erfolgt. Für eine möglichst einfache Konstruktion weist der Einsatzwagen einen Träger auf, an welchem der Verteileranschluss, die Aufnahmen sowie die Anschlüsse ausgebildet sind und welcher in betriebsbereitem Zustand im Reinigungsraum derart anordenbar ist, dass der Verteileranschluss des Einsatzwagens an den Versorgungsanschluss der Reinigungsvorrichtung angeschlossen ist.

Eine Fluidverbindung vom Verteileranschluss des Einsatzwagens zu den zugeordneten Anschlüssen kann z.B. auf einfache Weise durch Ausbildung des Trägers als Hohlprofil erreicht werden. Am Träger können z.B. auch Schienen der Aufnahmen für die Reinigungskörbe befestigt sein. Weiter können zudem am Träger rotierende Düsen zur Reinigung und Desinfektion von äusseren Bereichen des Instruments bzw. der Instrumente oder andere allenfalls erforderliche Anschlüsse oder Elemente der Reinigungsvorrichtung vorgesehen sein.

Die Erfindung betrifft auch ein Verfahren zum Reinigen von Instrumenten mit wenigstens einem zu reinigenden Lumen, insbesondere chirurgischen Instrumenten, vorzugsweise Endoskopen zur Durchführung auf einer Reinigungsvorrichtung mit einem Reinigungsraum, insbesondere einer erfindungsgemässen Reinigungsvorrichtung. Bei der Durchführung des Verfahrens kommt eine Verbindungsvorrichtung zum Einsatz, insbesondere eine vorliegend beschriebene Verbindungsvorrichtung, welche bevorzugt in einem Reinigungskorb abgeordnet ist, insbesondere einem vorliegend beschriebenen Reinigungskorb. Das Verfahren umfasst einen Reinigungsschritt, in welchem wenigstens ein Fluidparameter des flüssigen Mediums im Innenraum der Verbindungsvorrichtung von einem externen Überwachungssensor der Reinigungsvorrichtung überwacht wird, wobei insbesondere ein Überschreiten und/oder Unterschreiten eines vorgegebenen Minimalwerts des Fluidparameters überwacht wird und vorzugsweise der Fluidparameter ein Druck ist.

Zur Überwachung umfasst das Überwachen des wenigstens einen Fluidparameters vorzugsweise das Herstellen einer Wechselwirkung mit dem externen Überwachungssensor der Reinigungsvorrichtung durch eine entsprechend ausgebildete Einrichtung der Verbindungsvorrichtung.

Weiter kann das Verfahren einen Vorbereitungsschritt umfassen, in welchem der Ausgangsanschluss der Verbindungsvorrichtung direkt oder indirekt an das zu reinigende Lumen des Instruments angeschlossen wird, und einen Anschlussschritt, in welchem der Eingangsanschluss der Verbindungsvorrichtung direkt oder indirekt an einen Versorgungsanschluss der Reinigungsvorrichtung angeschlossen und die Verbindungsvorrichtung mit der Einrichtung bei dem Überwachungssensor angeordnet wird.

Bei einer in einem Reinigungskorb angeordneten Verbindungsvorrichtung wird im Vorbereitungsschritt das Instrument in einem Aufnahmebereich des Reinigungskorbs angeordnet. Zur Durchführung des Anschlussschritts wird der Reinigungskorb in eine entsprechende Aufnahme der Reinigungsvorrichtung eingebracht, insbesondere in eine Aufnahme einer Reinigungsvorrichtung mit Aufnahmen wie vorliegend beschrieben. Bevorzugt sind der Reinigungskorb und die Reinigungsvorrichtung derart ausgebildet, dass während des Anschlussschritts keine Manipulation des Instruments erforderlich ist.

Aus der nachfolgenden Detailbeschreibung und der Gesamtheit der Patentansprüche ergeben sich weitere vorteilhafte Ausführungsformen und Merkmalskombinationen der Erfindung.

### Beschreibung der Zeichnungen

Die zur Erläuterung der Ausführungsbeispiele verwendeten Zeichnungen zeigen schematisch:
- Fig. 1: Äussere Schrägansicht eines Reinigungskorbs mit einer erfindungsgemässen Verbindungsvorrichtung;
- Fig. 2: Ausschnitt eines Schnittansicht der Verbindungsvorrichtung in einer zu einer Grundfläche des Reinigungskorbs parallelen Ebene;
- Fig. 3: Ausschnitt einer äusseren Schrägansicht der Verbindungsvorrichtung im Reinigungskorb gemäss Fig. 1;
- Fig. 4: Ausschnitt einer Schnittansicht der Verbindungsvorrichtung in einer zu einem hinteren Wandabschnitt des Reinigungskorbs parallelen Ebene;
- Fig. 5: Schnittansicht in der Ebene der Fig. 4 durch eine gesamte Reinigungsvorrichtung mit Einsatzwagen und Reinigungskorb;
- Fig. 6: Ausschnitt einer Schnittansicht der Verbindungsvorrichtung in einer zu einem seitlichen Wandabschnitt des Reinigungskorbs parallelen Ebene mit einem auf einen Anschlussstutzen aufgesetzten Kopplungselement;
- Fig. 7: eine alternative Ausführungsform einer erfindungsgemässen Verbindungsvorrichtung mit einer Einrichtung ohne bewegliche mechanische Teile.

Grundsätzlich sind in den Figuren einander entsprechende Teile mit gleichen Bezugszeichen versehen.

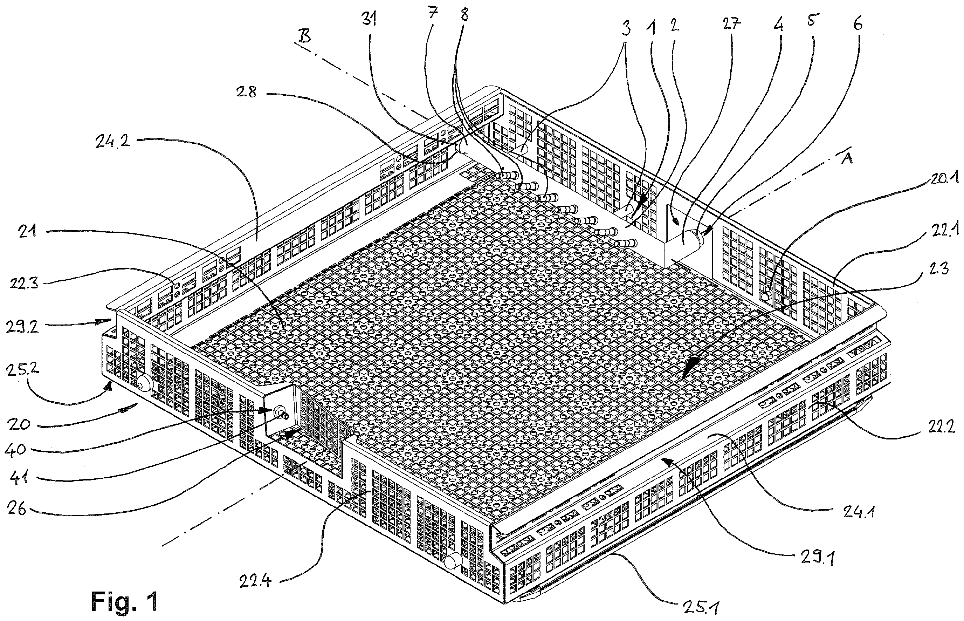

Figur 1 zeigt eine äussere Schrägansicht eines Reinigungskorbs 20 mit einer erfindungsgemässen Verbindungsvorrichtung 1. Der Reinigungskorb 20 weist eine rechteckige Grundfläche 21 auf, an deren 4 Seiten jeweils ein Wandabschnitt 22.1 bis 22.4 anschliesst. Die Wandabschnitte 22.1 bis 22.4 umfassen eine Rückwand 22.1, zwei Seitenwände 22.2 und 22.3 sowie eine Stirnwand 22.4. Rückwand 22.1 und Stirnwand 22.4 liegen sich gegenüber, wobei die Rückwand 22.1 bei betriebsbereiter Anordnung in einem Reinigungsraum 51 einer Reinigungsvorrichtung 50 bei einer Rückwand 52 des Reinigungsraums 51 angeordnet ist. Die Rückwand 22.1 ist somit in Einschubrichtung des Reinigungskorbs 10 angeordnet. Die Einschubrichtung ist dabei parallel zu den Seitenwänden 22.2 und 22.3 und der Grundfläche 21 ausgerichtet. Ein mittig zwischen den Seitenwänden 22.2 und 22.3 parallel zur Einschubrichtung angeordnete gedachte Achse A ist im Folgenden als Längsachse A des Reinigungskorbs 20 bezeichnet. Grundfläche 21 sowie Wandabschnitte 22 begrenzen einen Aufnahmebereich 23 für ein zu reinigendes Instrument. Die Längsachse A ist derart von der Grundfläche 21 beabstandet angeordnet, dass sie in einer zur Grundfläche 21 parallelen Ebene C der Verbindungsvorrichtung 1 liegt.

Grundfläche 21 sowie Wandabschnitte 22 sind siebartig mit einer Vielzahl von Durchbrüchen versehen, sodass das flüssige Medium aus dem Aufnahmebereich 23 abfliessen kann. Die Durchbrüche sind gemäss einem Raster angeordnet, welcher gleichzeitig zur modularen Verankerung von Halterungselementen (nicht dargestellt) dient. Die Halterungselemente können beispielsweise über Rastelemente in das Raster eingeschnappt werden, sodass eine gute Halterung des Instruments im Aufnahmebereich 23 gewährleistet ist.

Die Seitenwände 22.2 und 22.3 sind jeweils als Profil ausgebildet, wobei eine über die gesamte Länge der jeweiligen Seitenwand 22.2 bzw. 22.3 sich erstreckende U-förmige Längsnut 29.1 bzw. 29.2 als Griff dient. An den vorgesehenen Stellen zum Tragen des Reinigungskorbs 20 durch einen Benutzer weisen die Seitenwände 22.2 und 22.3 im Bereich der Nuten 29.1 und 29.2 zur optischen Abgrenzung durchgängige Bereiche 24.2 und 24.3 ohne Durchbrüche auf.

An einer Unterseite der Grundfläche 21 sind im Bereich der Seitenwände 22.2 und 22.3 in Längsrichtung verlaufende Gleitschienen 25.1 und 25.2 ausgebildet. Die Gleitschienen 25.1 und 25.2 bilden eine definierte Auflage des Reinigungskorbs 20, beispielsweise zum Gleiten auf entsprechenden Schienen einer Aufnahme für den Reinigungskorb 20. Die Gleitschienen 25.1 und 25.2 können beispielsweise aus einem Kunststoff gefertigt sein, während die Grundfläche 21 und Wandabschnitte 22 des Reinigungskorbs bevorzugt aus Metall gefertigt sind.

An der Stirnwand 22.4 ist eine Einbuchtung 26 ausgebildet, in welcher eine Anschlussvorrichtung 40 angeordnet ist. Die Anschlussvorrichtung 40 umfasst einen bezüglich der Stirnwand 22.4 aussenseitig angeordneten Eingangsanschluss 41, welcher zum Anschluss einer Dichtigkeitsmessvorrichtung der Reinigungsvorrichtung 50 vorgesehen ist. Zum Aufnahmebereich 23 hin gerichtet ist innenseitig an der Stirnwand 22.4 ein Ausgangsanschluss vorhanden (nicht sichtbar), welcher zum Anschluss einer auf Dichtigkeit zu prüfenden Kavität des Instruments vorgesehen ist. Die Anschlüsse sind als Anschlussstutzen ausgebildet und können herkömmliche, z.B. genormte, Kupplungen zum Herstellen einer luft- und/oder fluiddichten Verbindung umfassen.

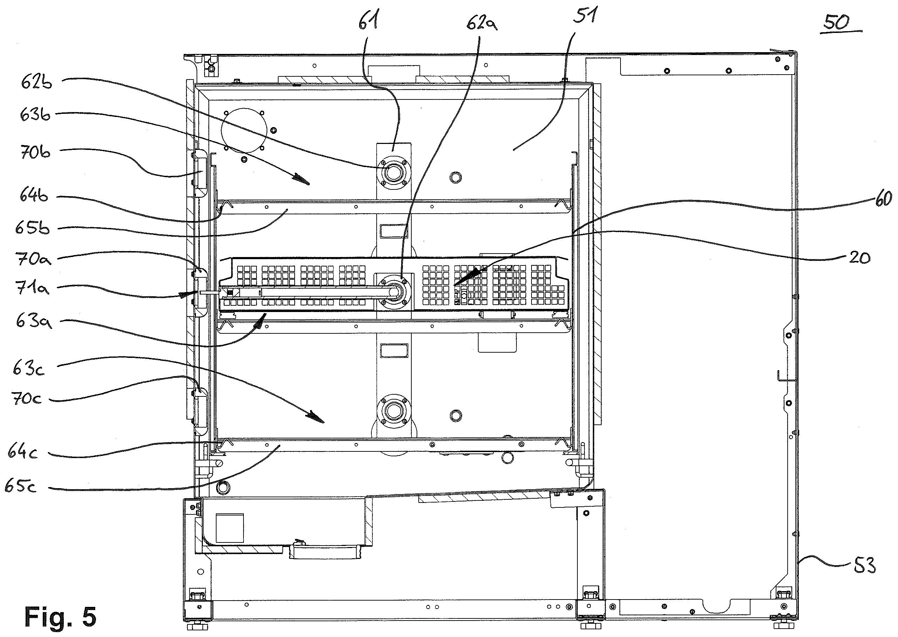

In einem Bereich bei der Rückwand 22.1 ist die Verbindungsvorrichtung 1 im Aufnahmebereich 23 angeordnet. Die Verbindungsvorrichtung 1 umfasst einen ersten Rohrabschnitt 2 mit kreiszylindrischem Querschnitt (siehe auch Fig. 5). Der Rohrabschnitt 2 ist mit seiner Längsachse B weitgehend senkrecht zur Längsachse A und parallel zur Grundfläche 21 des Reinigungskorbs 20 angeordnet. Der Rohrabschnitt 2 ist über zwei Haltebolzen 3 von der Rückwand 22.1 beabstandet an dieser befestigt. Am Rohrabschnitt 2 sind mehrere Anschlussstutzen 8 zum direkten oder indirekten Anschluss eines oder mehrerer Lumen des Instruments ausgebildet, welche sich in Richtung von der Rückwand 22.1 und von der Grundfläche 21 weg, schräg nach oben ragend in den Aufnahmebereich 23 erstrecken. Die Anschlussstutzen 8 sind auf einer Mantellinie des Rohrabschnitts 2 angeordnet und kommunizieren mit einem Innenraum 9 der Verbindungsvorrichtung 1. Der Rohrabschnitt 2 erstreckt sich dabei von der Seitenwand 22.3 bis hin zu einer Mitte zwischen den Seitenwänden 22.2 und 22.3, wo ein weiterer Rohrabschnitt 4 weitgehend senkrecht an den Rohrabschnitt 2 anschliesst. Eine Längsachse des Rohrabschnitts 4 fällt dabei mit der Längsachse A des Reinigungskorbs 20 zusammen. Die Achsen A und B spannen somit die oben genannte Ebene C der Verbindungsvorrichtung 1 auf.

Der Rohrabschnitt 4 erstreckt sich ausgehend vom Rohrabschnitt 2 zur Rückwand 22.1 hin und ragt mit einem Längsende 5 durch einen an der Rückwand 22.1 ausgebildeten Durchbruch 27 hindurch. Am Längsende 5 ist ein Eingangsanschluss 6 der Verbindungsvorrichtung 1 ausgebildet, mit welchem die Verbindungsvorrichtung 1 an einen Versorgungsanschluss anschliessbar ist. Der Durchbruch 27 ist dabei derart ausgebildet, dass der Versorgungsanschluss, welcher in betriebsbereitem Zustand das Längsende 5 fluiddicht umschliesst, im Durchbruch 27 anordenbar ist. Am Eingangsanschluss 6 weist der Rohrabschnitt 4 einen konisch zulaufenden Endbereich auf, um ein Einführen in eine Öffnung des Versorgungsanschlusses zu vereinfachen.

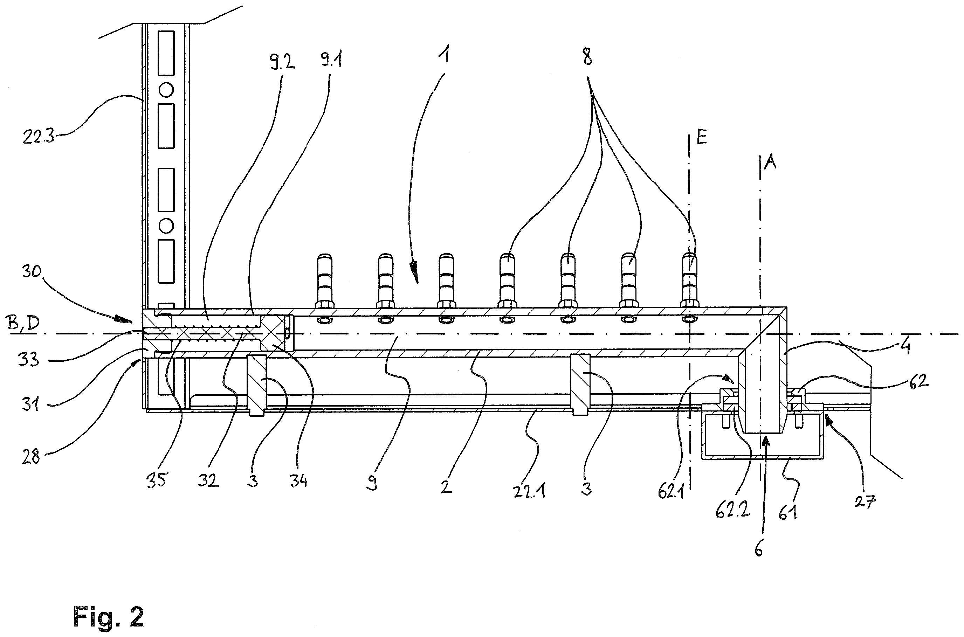

An der Seitenwand 22.3 ist der Rohrabschnitt 2 mit seinem Längsende 7 ebenfalls in einem in der Seitenwand 22.3 ausgebildeten Durchbruch 28 angeordnet. Am Längsende 7 ist im Rohrabschnitt 2 eine Einrichtung 30 für eine Überwachung wenigstens eines Fluidparameters des flüssigen Mediums im Innenraum 9 der Verbindungsvorrichtung 1 durch einen externen Sensor 70a der Reinigungsvorrichtung 50 vorhanden (siehe unten). Die Einrichtung 30 ist im Folgenden anhand der Fig. 2 näher beschrieben.

Aussenseitig an der Rückwand 22.1 ist ein Signalmittel 20.1 angebracht, welches vorliegend als einfacher Permanentmagnet ausgebildet ist. Ein entsprechender Präsenzdetektor an der Rückwand 52 des Reinigungsraums 51 kann auf diese Weise eine Präsenz eines Reinigungskorbs detektieren. Bei z.B. mehreren Aufnahmen (siehe unten) im Reinigungsraum 51 ist jeder Aufnahme ein derartiger Präsenzdetektor zugeordnet.

Figur 2 zeigt einen Ausschnitt einer Schnittansicht der Verbindungsvorrichtung 1 in der Ebene C von der Grundfläche 21 des Reinigungskorbs 20 her gesehen. In der Ansicht der Fig. 2 ist ein Versorgungsanschluss 62a dargestellt, welcher z.B. an einem Einsatzwagen 60 oder direkt im Reinigungsraum 51 der Reinigungsvorrichtung 50 ausgebildet ist (siehe hierzu auch Fig. 5). Der Versorgungsanschluss 62a ist im Durchbruch 27 angeordnet, wobei der Eingangsanschluss 6 der Verbindungsvorrichtung 1 in eine Anschlussöffnung 62.1 des Versorgungsanschlusses 62a fluiddicht eingreift. Die Anschlussöffnung 62.1 kommuniziert mit dem Innenraum eines hohlen Trägers 61 des Einsatzwagens 60. Die Anschlussöffnung 62.1 weist hierzu eine ringförmige Dichtlippe 62.2 auf, welche das Längsende 5 des Rohrabschnitts 4 fluiddicht umschliesst.

Der Versorgungsanschluss 62a ist bevorzugt selbstdichtend ausgebildet. Bei nicht in die Anschlussöffnung 62.1 eingreifendem Eingangsanschluss 6 ist die Anschlussöffnung 62.1 von innen her mit einer Dichtzunge (nicht dargestellt) verschlossen. Ein Flüssigkeitsdruck in der Zuführung zum Versorgungsanschluss 62a drückt die Dichtzunge dichtend gegen die Dichtlippe 62.2. Beim Einführen des Eingangsanschlusses 6 wird die Dichtzunge von der Dichtlippe 62.2 abgehoben, womit das flüssige Medium in den Eingangsanschluss 6 eintreten kann.

Der Durchbruch 28 für das Längsende 7 des Rohrabschnitts 2 ist derart bemessen, dass ein Abschlussstück 31 der Einrichtung 30, mit welchem der Rohrabschnitt 2 am Längsende 7 verschlossen ist, den Durchbruch 28 im Wesentlichen ausfüllt. Eine äussere Stirnseite des Abschlussstücks 31 schliesst dabei mit der Aussenfläche der Seitenwand 22.3 bündig ab.

Das Abschlussstück 31 kann beispielsweise mit einem Aussengewinde in ein Innengewinde des Rohrabschnitts 2 eingeschraubt sein und liegt mit einem flanschartigen Kragen am Ende des Rohres an. Im Abschlussstück 31 ist ein zentraler Durchbruch ausgebildet, in welchem ein in Längsrichtung B angeordneter Betätigungsstift 33 eines als Stössel ausgebildeten Betätigungselements 32 in Längsrichtung B verschiebbar geführt gelagert ist. Im Innenraum 9 ist ein Kolben 34 des Betätigungselements 32 angeordnet, welcher fest mit dem Betätigungsstift 33 verbunden und ebenfalls in Längsrichtung B verschiebbar geführt gelagert ist. Hierzu weist vorliegend der Innenraum 9 einen entsprechend dem Durchmesser des Kolbens 34 aufgeweiteten Abschnitt 9.1 auf. Der Abschnitt 9.1 begrenzt dabei einen Hub des Kolbens 34 auf einer vom Abschlussstück 31 abgewandten Seite, wobei hierzu zusätzlich oder ersatzweise z.B. ein Anschlagstift im Innenraum 9 vorgesehen sein kann.

Der Kolben 34 ist über eine Schraubenfeder 35 am Abschlussstück 31 abgestützt, wobei die Schraubenfeder 35 den Betätigungsstift 33 umwindet, welcher somit gleichzeitig eine Führung für die Feder 35 bildet. In der Darstellung der Fig. 2 ist der Betätigungsstift 33 vollständig eingefahren, d.h. ein freies Längsende des Betätigungsstifts 33 schliesst weitgehend mit der äusseren Stirnseite des Abschlussstücks 31 ab. Der Kolben 34 ist dabei möglichst weit im Führungsbereich 9.1 in den Innenraum 9 hinein verschoben. Dies entspricht einer Ruheposition des Betätigungselements 32 wenn kein oder ein zu geringer Druck eines flüssigen Mediums im Innenraum 9 auf den Kolben 34 wirkt.

Um eine für eine Verschiebung des Kolbens 34 erforderliche Druckdifferenz in Längsrichtung B zu beiden Seiten des Kolbens 34 sicherzustellen, weist das Abschlussstück 31 neben dem genannten Durchbruch zur Führung des Betätigungsstifts 33 einen weiteren Durchbruch auf (nicht dargestellt), welcher einen Druckausgleich eines Innenraumabschnitts 9.2 zwischen Kolben 34 und Abschlussstück 31 mit der Aussenumgebung erlaubt. Alternativ kann auch eine entsprechende Öffnung im Rohrabschnitt 2 im Bereich de Innenraumabschnitts 9.2 aufweisen.

Die Federkraft der Schraubenfeder 35 ist derart bemessen, dass, bei freien Anschlussstutzen 8 (d.h. unverschlossen und ohne angeschlossene Lumen) und aufgrund des Druck eines über den Versorgungsanschluss 62a bereitgestellten flüssigen Mediums, der Kolben 34 gegen die Federkraft zum Abschlussstück 31 hin verschoben ist. Der Betätigungsstift 33 ist in diesem Fall ausgefahren und ragt über eine Aussenfläche des Abschlussstücks 31 hinaus; insbesondere über eine Aussenfläche der Seitenwand 22.3. Diese Position entspricht einer Betätigungsstellung des Betätigungselements 32.

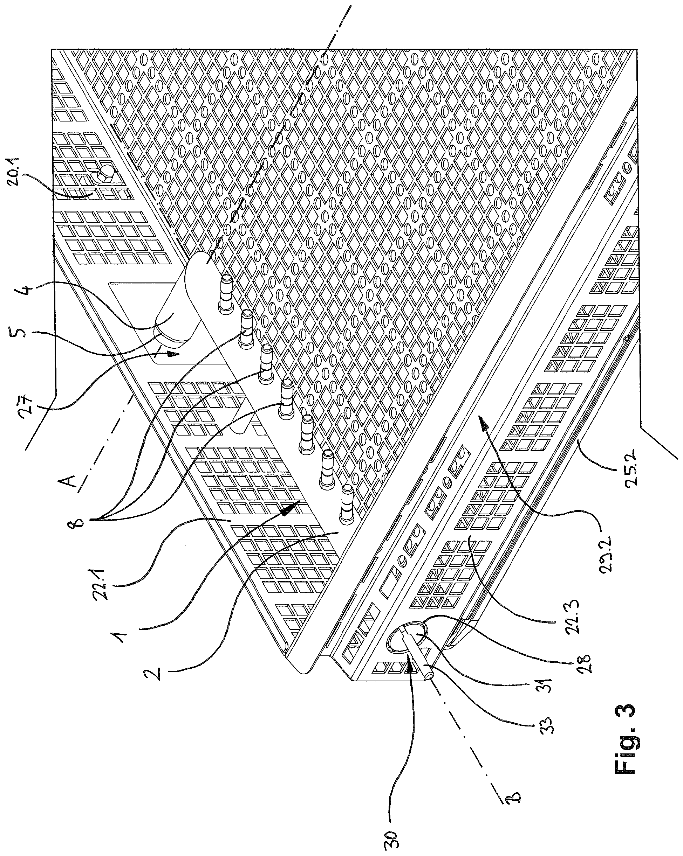

Figur 3 zeigt eine Detailansicht der Verbindungsvorrichtung 1 in einer äusseren Schrägansicht, wobei sich das Betätigungselement 32 in der Betätigungsstellung befindet und über die äussere Stirnseite des Abschlussstücks 31, insbesondere auch über die Aussenfläche der Seitenwand 22.3, hinausragt. Der herausragende Betätigungsstift 33 signalisiert somit aufgrund der Verschiebestellung, dass über das flüssige Medium ein Druck im Innenraum 9 auf den Kolben 34 wirkt (auf eine Darstellung des Versorgungsanschlusses ist in Fig. 3 verzichtet).

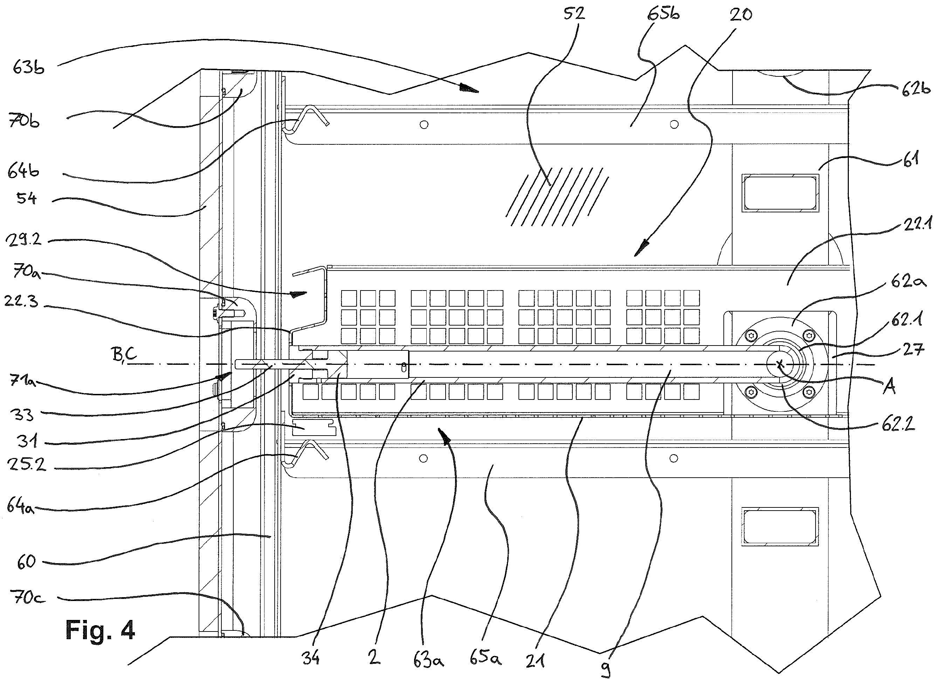

Figur 4 zeigt einen Ausschnitt einer Schnittansicht der Verbindungsvorrichtung 1 in einer zu einem hinteren Wandabschnitt 22.1 des Reinigungskorbs 20 parallelen Ebene D, in welcher die Längsrichtung B liegt. Ebene D steht dabei senkrecht auf der Längsachse A des Reinigungskorbs 20.

Der Reinigungskorb 20 ist in einer Aufnahme 63a des Einsatzwagens 60 angeordnet, welcher wiederum im Reinigungsraum 51 der Reinigungsvorrichtung 50 eingebracht ist. Die Aufnahme 63a weist Schienen 64a auf, auf welchen der Reinigungskorb 20 mit seinen Schienen 25.2 unterstützt ist und nach Art einer Schublade gleitend in Einschubrichtung, d.h. vorliegend in Richtung von A, verschiebbar ist. Die Schienen 64a der Aufnahme 63a sind über Streben 65a mit dem Träger 61 des Einsatzwagens 60 verbunden.

Die Rückwand 22.1 des in der Aufnahme 63a angeordneten Reinigungskorbs 20 ist bei der Rückwand 52 des Reinigungsraums 51 angeordnet. Der Eingangsanschluss 6 der Verbindungsvorrichtung 1 greift dabei in einen dem Versorgungsanschluss 62a entsprechenden Versorgungsanschluss 62a des Trägers 61 ein. Der Träger 61 wiederum ist über einen eigenen Eingangsanschluss (nicht sichtbar) an einen zentralen Versorgungsanschluss (nicht sichtbar) der Reinigungsvorrichtung 50 angeschlossen, welche das flüssige Medium über den Eingangsanschluss dem hohlen Träger 61 zuführt. Der hohle Träger 61 wirkt somit als Zuführungsvorrichtung, welche die über den Eingangsanschluss des Trägers 61 eingebrachte Flüssigkeit dem Versorgungsanschluss 62a (respektive auch Versorgungsanschluss 62b der Aufnahme 63b etc., siehe unten) zuführt.

Der Einsatzwagen 60 kann eine weitere Aufnahme 63b etc. für einen weiteren Reinigungskorb 20 aufweisen. Die Aufnahme 63b ist weitgehend identisch ausgestaltet und weist ebenfalls einen zugeordneten Versorgungsanschluss 62b am Träger 61 auf (siehe auch Fig. 5).

Der Einsatzwagen 60 kann aus dem Reinigungsraum 51 ausgebracht werden bzw. auf eine Verschlussklappe des Reinigungsraums 51 ausgefahren werden, sodass der oder die Reinigungskörbe 20 einfacher in die Aufnahmen 63a bzw. 63b eingesetzt werden können. Wird der Einsatzwagen 60 in den Reinigungsraum 51 eingebracht, wird automatisch über den Verteileranschluss des Trägers 61 ein Anschluss mit einem Versorgungsanschluss der Reinigungsvorrichtung 50 hergestellt.

In der Darstellung der Fig. 4 ist der Innenraum 9 über den Versorgungsanschluss 62a des Trägers 61 mit dem flüssigen Medium (nicht dargestellt) wie beispielsweise einer Reinigungsflüssigkeit und/oder einer Desinfektionsflüssigkeit beschickt. Der Kolben 34 ist aufgrund des so auf ihn ausgeübten Drucks weitgehend vollständig zum Abschlussstück 31 hin verschoben. Das Betätigungselement 32 befindet sich somit in der Betätigungsstellung und ragt mit dem Betätigungsstift 33 über die Aussenfläche der Seitenwand 22.3 hinaus. Die Feder 35 ist weitgehend vollständig komprimiert.

Der Reinigungskorb 20 ist in der Aufnahme mit seiner Seitenwand 22.3 benachbart zu einer Seitenwand 54 des Reinigungsraums 51 angeordnet. An der Seitenwand 54 ist ein mit einem Messbereich 71a dem Reinigungsraum 51 zugewandter Drucksensor 70a angebracht. Der Drucksensor 70a kann z.B. als einfacher Druckschalter ausgebildet sein. Der Drucksensor 70a ist der Aufnahme 63a derart zugeordnet, dass die Verbindungsvorrichtung 1 mit der Einrichtung 30 dem Messbereich 71a des Drucksensors 70a vorgelagert ist. Insbesondere ist der Drucksensor 70a derart bezüglich der Aufnahme 63a angeordnet, dass der Betätigungsstift 33 in der Betätigungsstellung des Betätigungselements 32 in eine Wirkverbindung mit dem Messbereich 71a tritt. Insbesondere kann der Drucksensor 70a vom Betätigungsstift 33 in der Betätigungsstellung betätigt werden, sodass über den Drucksensor 70a ein Druck im Innenraum 9 gemessen und an eine Steuerungsvorrichtung (nicht dargestellt) der Reinigungsvorrichtung 50 signalisiert werden kann. Insbesondere kann im Falle, dass der Drucksensor 70a als Druckschalter ausgebildet ist, bei ausreichendem Druck auf den Kolben 34 eine entsprechend grosse Druckkraft über den Betätigungsstift 33 auf den Druckschalter 70a ausgeübt werden, sodass der Druckschalter 70a schaltet. Je nach Einstellung der erforderlichen Schaltkraft des Druckschalters 70a kann somit das Überschreiten eines vorgegeben Minimalwerts eines im Innenraum 9 vorhandenen Drucks des flüssigen Mediums gemessen werden.

Die Wirkverbindung ist somit während des Reinigungsvorgangs nicht per se beständig, sondern erfolgt nur, wenn ein Druck im Innenraum 9 wirkt. Es versteht sich, dass der Aufnahme 63b bzw. jeder weiteren Aufnahme (z.B. 63c, siehe unten) entsprechend ein an der Seitenwand 54 angebrachter Drucksensor 70b (bzw. 70c) gleichartig zugeordnet ist.
Fig. 5 zeigt eine Gesamtansicht der Schnittansicht der Fig. 4 durch die gesamte Reinigungsvorrichtung 50. Der Einsatzwagen 60 weist drei übereinander angeordnete Aufnahmen 63a, 63b und 63c für einen Reinigungskorb 20 auf. Jede der Aufnahmen 63a, 63b und 63c weist über Streben 65a, 65b und 65c mit dem in Stapelrichtung ausgerichteten Träger 61 verbundene Schienen 64a, 64b und 64c zur Unterstützung jeweils eines Reinigungskorbes 20 auf. In der Darstellung der Fig. 5 ist nur die mittlere Aufnahme 63a mit dem Reinigungskorb 20 versehen.

Wie aus Fig. 5 ersichtlich, ist jeder Aufnahme ein Drucksensor 70a, 70b bzw. 70c analog der in Fig. 4 beschriebenen Anordnung zugeordnet. Ebenso ist jeder Aufnahme 63a, 63b und 63c ein am Träger 61 ausgebildeter Versorgungsanschluss 62a, 62b und 62c zugeordnet.

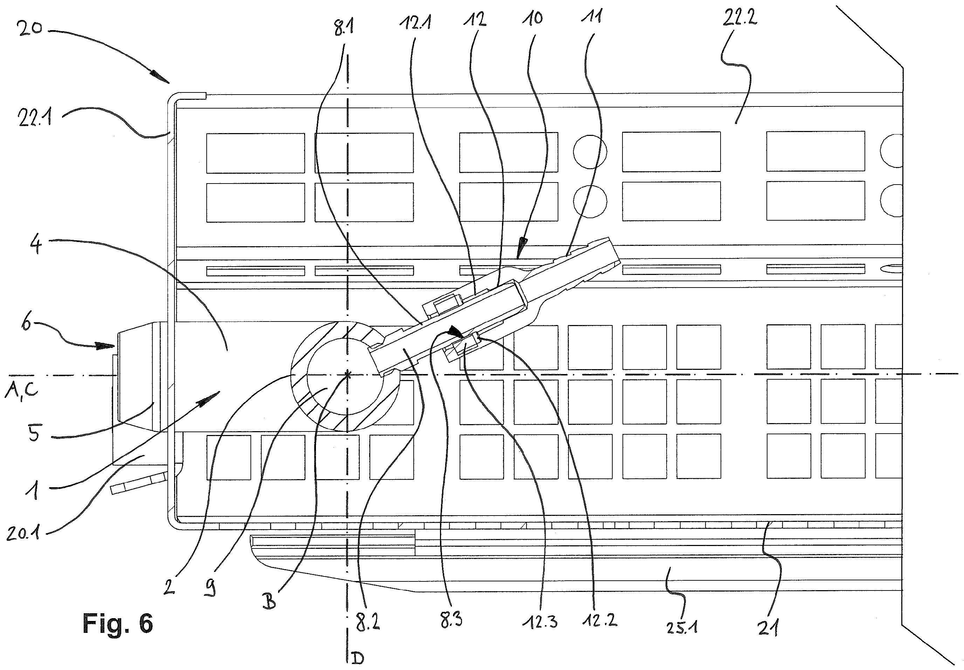

Figur 6 zeigt einen Querschnitt in einer zur Seitenwand 22.3 parallelen Ebene E, welche senkrecht auf der Richtung B steht und durch einen (8.1) der Anschlussstutzen 8 geht. Der Anschlussstutzen 8.1 ist vorliegend als separates Teil ausgebildet und in eine entsprechende Öffnung am Rohrabschnitt 2 eingeschraubt.

Der Anschlussstutzen 8.1 weist in seiner Längsrichtung einen innen liegenden Fluidkanal 8.2 auf, welcher mit dem Innenraum 9 der Verbindungsvorrichtung 1 kommuniziert. Aussenseitig auf seiner Mantelfläche weist der Anschlussstutzen 8.1 eine umlaufende Nut 8.3 auf. Auf den Anschlussstutzen 8.1 ist ein Kopplungselement 10 aufgesetzt, welches zum vereinfachten und sicheren Anschluss eines Lumens des zu reinigenden Instruments dient.

Das Kopplungselement 10 umfasst einen Anschlussbereich 11, auf welchen z.B. ein das Lumen unfassender Schlauch aufgesteckt werden kann. Der Anschlussbereich 11 weist hierzu auf bekannte Weise mantelseitig umlaufende, bezüglich einer Aufsteckrichtung des Schlauchs hinterschnittene Kerben auf, um die Verbindung zu sichern.

Ein Kopplungsbereich 12 des Kopplungselements 10 umfasst einen Aufnahmeraum 12.1, in welchem der Anschlussstutzen 8.1 zumindest teilweise aufnehmbar ist. Im Aufnahmeraum 12.1 ist innenseitig eine umlaufende Nut 12.2 ausgebildet, in welcher ein ringförmiges elastisches Dichtelement 12.3 angeordnet ist. In auf den Anschlussstutzen 8.1 aufgesetztem Zustand sind die Nut 8.3 und das Dichtelement 12.3 derart zueinander angeordnet, dass das Dichtelement 12.3 in die Nut 8.3 eingreift. Das Dichtelement 12.3 erfüllt dabei gleichzeitig zwei Funktionen: Zum einen wird die so hergestellte Anschlussverbindung fluiddicht abgedichtet. Zum anderen arretiert das Dichtelement 12.3 durch den Eingriff in die Nut 8.3 am Anschlussstutzen 8.1 das Kopplungselement 10 am Anschlussstutzen und sichert die Verbindung so gegen ein ungewolltes Abziehen des Kopplungselements 10. Das Kopplungselement 10 stellt somit eine einfache und kostengünstige Verbindung bereit, welche einen sicheren und fluiddichten Anschluss eines Schlauchs an einen entsprechend ausgebildeten Anschlussstutzen erlaubt.

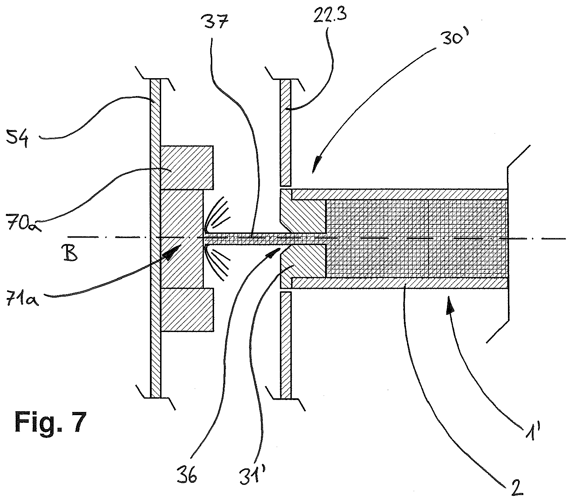

Figur 7 zeigt eine alternative Ausführungsform einer Verbindungsvorrichtung 1' mit einer Einrichtung 30' ohne bewegliche mechanische Teile. Der Rohrabschnitt 2 der Verbindungsvorrichtung 1' ist endseitig mit einem Abschlussstück 31' verschlossen. Im Abschlussstück 31' ist eine Düsenöffnung 36 ausgebildet, durch welche das unter Druck stehende flüssige Medium im Innenraum 9 der Verbindungsvorrichtung 1' als gerichteter Strahl 37 austreten kann. In betriebsbereitem Zustand ist die Einrichtung 30' derart vor dem Messbereich 71a des an der Seitenwand 54 des Reinigungsraums 51 angebrachten Drucksensors 70a angeordnet, dass der austretende Strahl 37 auf den Messbereich 71a auftrifft. Aufgrund der so über den Strahl 37 ausgeübten Druckkraft auf den Messbereich 71a des Drucksensors 70a, kann auf einen Druck im Innenraum 9 geschlossen werden. Insbesondere kann bei einer Ausbildung des Drucksensors 70a als Druckschalter auf einfache Weise das Überschreiten eines vorgegebenen Minimaldrucks registriert werden. Diese Ausführungsform hat den Vorteil, dass keine mechanisch beweglichen Teile erforderlich sind und somit eine besonders robuste und einfache Variante der Erfindung bereitgestellt ist.

## Patentansprüche

1. Reinigungsvorrichtung (50) mit einem Reinigungsraum (51) zur Reinigung von Instrumenten mit wenigstens einem zu reinigenden Lumen, wobei wenigstens ein Versorgungsanschluss vorhanden ist, über welchen ein flüssiges Medium bereitstellbar ist, und welcher zum Anschluss einer Verbindungsvorrichtung (1, 1') ausgebildet ist, wobei die Verbindungsvorrichtung (1, 1') einen Innenraum (9) mit einem Eingangsanschluss (6) und wenigstens einem Ausgangsanschluss (8) aufweist, wobei der Eingangsanschluss (6) an den Versorgungsanschluss der Reinigungsvorrichtung (50) anschliessbar ist und der Ausgangsanschluss (8) an das zu reinigende Lumen des Instruments anschliessbar ist, wobei wenigstens ein Überwachungssensor (70a) vorhanden ist, dass zur Überwachung wenigstens eines Fluidparameters eines flüssigen Mediums im Innenraum (9) der Verbindungsvorrichtung (1, 1') über eine Einrichtung (30, 30') der Verbindungsvorrichtung (1, 1') eine Wechselwirkung des flüssigen Mediums mit dem Überwachungssensor (70a) der Reinigungsvorrichtung (50) herstellbar ist, wenn die Verbindungsvorrichtung (1, 1') in betriebsbereitem Zustand an den wenigstens einen Versorgungsanschluss (62a) der Reinigungsvorrichtung (50) angeschlossen ist, **dadurch gekennzeichnet, dass** der Überwachungssensor (70a) fest im Reinigungsraum (51) angeordnet ist, und dass die Wechselwirkung des flüssigen Mediums mit dem Überwachungssensor (70a) über eine nicht beständige Verbindung herstellbar ist, wobei die Einrichtung (30) einen gegen eine Rückstellkraft verschiebbar im Innenraum (9) gelagerten Stössel (32) umfasst, welcher aufgrund des wenigstens einen Fluidparameters unterschiedliche Verschiebestellung einnehmen kann, in welchem er mit einem Betätigungsstift (33) unterschiedlich weit aus der Verbindungsvorrichtung (1, 1') herausragt, um die Wechselwirkung mit dem externen Überwachungssensor (70a) zu ermöglichen oder die Einrichtung (30') eine Düsenöffnung (36) umfasst, durch welche das im Innenraum (9) vorhandene flüssige Medium als gerichteter Strahl (37) aus dem Innenraum (9) austreten kann, um die Wechselwirkung mit dem externen Überwachungssensor (70a) zu ermöglichen.

2. Reinigungsvorrichtung (50) gemäss Anspruch 1, **dadurch gekennzeichnet, dass** der wenigstens eine Fluidparameter ein Druck ist.

3. Reinigungsvorrichtung (50) gemäss Anspruch 1 oder 2, **dadurch gekennzeichnet, dass** die Einrichtung (30, 30') der Verbindungsvorrichtung (1, 1') derart ausgebildet ist, dass sie die Wechselwirkung nur bei Überschreiten und/oder Unterschreiten eines Minimalwerts des überwachten Fluidparameters ermöglicht.

4. Reinigungsvorrichtung (50) gemäss Anspruch 1, 2 oder 3, **dadurch gekennzeichnet, dass** die Wechselwirkung eine mechanische Wirkverbindung ist.

5. Reinigungsvorrichtung (50) gemäss Anspruch 4, **dadurch gekennzeichnet, dass** die mechanische Wirkverbindung eine Druckkraft ist.

6. Reinigungsvorrichtung (50) gemäss Anspruch 4, **dadurch gekennzeichnet, dass** mehrere Ausgangsanschlüsse (8) zum Anschluss mehrerer zu reinigender Lumen eines oder mehrerer Instrumente vorhanden sind.

7. Reinigungsvorrichtung (50) gemäss einem der Ansprüche 1 bis 6, **dadurch gekennzeichnet, dass** die Verbindungsvorrichtung (1, 1') wenigstens einen länglichen Rohrabschnitt (2) umfasst, in welchem der Innenraum (9) ausgebildet ist und an dessen einen Längsende die Einrichtung (30, 30') angeordnet ist.

8. Reinigungsvorrichtung (50) gemäss Anspruch 7, **dadurch gekennzeichnet, dass** die Verbindungsvorrichtung (1, 1') einen weiteren länglichen Rohrabschnitt (4) umfasst, welcher mit einem seiner Längsenden weitgehend rechtwinklig an den wenigstens einen Rohrabschnitt (2) anschliesst, wobei am freien Ende (5) des weiteren Rohrabschnitts (4) der Eingangsanschluss (6) ausgebildet ist.

9. Reinigungsvorrichtung (50) gemäss einem der Ansprüche 1 bis 8, **dadurch gekennzeichnet, dass** der Versorgungsanschluss (62a) der Reinigungsvorrichtung (50) an einer Rückwand (52) des Reinigungsraums (51) und der Überwachungssensor (70a) an einer Seitenwand (54) des Reinigungsraums (51) angeordnet sind.

10. Reinigungsvorrichtung (50) gemäss einem der Ansprüche 1 bis 9, **dadurch gekennzeichnet, dass** der Überwachungssensor (70a) einen Druckschalter umfasst, welcher durch eine Druckkraft betätigbar ist.

11. Reinigungsvorrichtung (50) gemäss einem der Ansprüche 1 bis 10, **dadurch gekennzeichnet, dass** im Reinigungsraum (51) wenigsten eine Aufnahme (63a) zur Anordnung von einem Reinigungskorb (20) mit einem Aufnahmebereich (23) für ein Instrument mit einem zu reinigenden Lumen, wobei der Reinigungskorb (20) eine Verbindungsvorrichtung (1, 1') umfasst, vorhanden ist und dass in betriebsbereiter Anordnung des Reinigungskorbs (20) in der Aufnahme (63a) die Verbindungsvorrichtung (1, 1') betriebsbereit an den Versorgungsanschluss (62a) angeschlossen und die mit der Einrichtung bei einem Messbereich des Überwachungssensors (70a) angeordnet ist.

12. Reinigungsvorrichtung (50) gemäss Anspruch 11, **dadurch gekennzeichnet, dass** die Verbindungsvorrichtung (1, 1') fest mit dem Reinigungskorb (20) verbunden ist.

13. Reinigungsvorrichtung (50) gemäss Anspruch 11 oder 12, **dadurch gekennzeichnet, dass** die Verbindungsvorrichtung (1, 1') mit der Einrichtung (30, 30') und dem Eingangsanschluss (6) jeweils in einem Randbereich des Aufnahmebereichs (23) des Reinigungskorbs (20) angeordnet ist, sodass die Einrichtung (30, 30') und gegebenenfalls der Eingangsanschluss (6) von ausserhalb des Aufnahmebereichs (23) her zugänglich sind.

14. Reinigungsvorrichtung (50) gemäss einem der Ansprüche 11 bis 13, welcher schubladenartig mit einer weitgehend rechteckigen Grundfläche (21) ausgebildet ist und eine Umrandung mit mehreren Wandabschnitten (22.1-22.4) aufweist, sodass der Reinigungskorb (20) in einer entsprechend ausgebildeten Aufnahme (63a) eines Einsatzwagens (60) oder des Reinigungsraums (51) nach Art einer Schublade ein- und ausschiebbar ist.

15. Reinigungsvorrichtung (50) gemäss Anspruch 14, **dadurch gekennzeichnet, dass** die Wandabschnitte (22.1-22.4) und/oder die Grundfläche (21) ein vergleichsweise feinmaschiges Raster umfassen.

16. Reinigungsvorrichtung (50) gemäss Anspruch 14 oder 15, **dadurch gekennzeichnet, dass** die Verbindungseinrichtung (1, 1') mit dem Eingangsanschluss (6) bei einem hinteren Wandabschnitt (22.1) und mit der Einrichtung (30, 30') an einem seitlichen Wandabschnitt (22.3) angeordnet ist, wobei bei der Einrichtung (30,30') ein Durchbruch (28) im seitlichen Wandabschnitt (22.3) und bei dem Eingangsanschluss (6) ein Durchbruch (27) im hinteren Wandabschnitt (22.1) ausgebildet ist.

17. Reinigungsvorrichtung (50) gemäss einem der Ansprüche 11 bis 16, **dadurch gekennzeichnet, dass** ein Signalmittel (20.1) zur Signalisierung einer Präsenz des Reinigungskorbs (20) zur Detektierung durch einen entsprechenden Präsenzdetektor der Reinigungsvorrichtung (50) vorhanden ist.

18. Reinigungsvorrichtung (50) gemäss Anspruch 17, **dadurch gekennzeichnet, dass** das Signalmittel (20.1) ein passives magnetisches Signalmittel (20.1) ist.

19. Reinigungsvorrichtung (50) gemäss einem der Ansprüche 11 bis 18, **dadurch gekennzeichnet, dass** weitere Aufnahmen (63b, 63c) vorhanden sind, welchen analog der ersten Aufnahme (63a) jeweils ein Überwachungssensor (70b, 70c) zugeordnet ist, und dass jeder Aufnahme (63a, 63b, 63c) ein Anschluss (62a, 62b, 62c) zum Bereitstellen eines flüssigen Mediums zugeordnet ist, an welchen die jeweils zugehörige Verbindungsvorrichtung (1, 1') in betriebsbereiter Anordnung des Reinigungskorbs (20) in der jeweiligen Aufnahme (63a, 63b, 63c) betriebsbereit angeschlossen ist.

20. Reinigungsvorrichtung (50) gemäss einem der Ansprüche 11 bis 19, **dadurch gekennzeichnet, dass** die Reinigungsvorrichtung (50) einen Einsatzwagen (60) zur Anordnung im Reinigungsraum (51) umfasst, an welchem die wenigsten eine Aufnahme (63a) sowie der zugeordnete Anschluss (62a) ausgebildet ist, wobei der Einsatzwagen (60) einen Verteileranschluss aufweist, welcher an den Versorgungsanschluss der Reinigungsvorrichtung (50) anschliessbar ist und über einen Fluidkanal im Einsatzwagen (60) fluidführend mit dem zugeordneten Anschluss (62a) verbunden ist.

21. Reinigungsvorrichtung (50) gemäss Anspruch 20, **dadurch gekennzeichnet, dass** der Einsatzwagen (60) einen Träger (61) aufweist, an welchem der Verteileranschluss, die wenigstens eine Aufnahme (63a) sowie der zugeordnete Anschluss (62a) ausgebildet sind und welcher in betriebsbereitem Zustand im Reinigungsraum (51) derart anordenbar ist, dass der Verteileranschluss des Einsatzwagens (60) an den Versorgungsanschluss der Reinigungsvorrichtung (50) angeschlossen ist.

22. Verfahren zum Reinigen von Instrumenten mit wenigstens einem zu reinigenden Lumen, zur Durchführung auf einer Reinigungsvorrichtung gemäss einem der vorhergehenden Ansprüche, wobei im Reinigungsraum (51) eine Verbindungsvorrichtung zum Einsatz kommt, umfassend einen Reinigungsschritt, in welchem wenigstens ein Fluidparameter des flüssigen Mediums im Innenraum (9) der Verbindungsvorrichtung (1, 1') von einem Überwachungssensor (70a) überwacht wird, **dadurch gekennzeichnet, dass** der Überwachungssensor (70a) fest im Reinigungsraum (51) angeordnet ist und, dass das wenigstens ein Fluidparameter über eine nicht beständige Verbindung überwacht wird.

23. Verfahren nach Anspruch 22, **dadurch gekennzeichnet, dass** ein Überschreiten und/oder Unterschreiten eines vorgegebenen Minimalwerts des Fluidparameters überwacht wird.

24. Verfahren nach Anspruch 22 oder 23, **dadurch gekennzeichnet, dass** der Fluidparameter ein Druck ist.

## Claims

1. Cleaning device (50) with a cleaning chamber (51) for cleaning instruments with at least one lumen to be cleaned, wherein at least one supply connection is provided, via which a liquid medium can be made available, and which is designed for connection of a connecting device (1, 1'), wherein the connecting device (1, 1') has an interior space (9) with an inlet connection (6) and at least one outlet connection (8), wherein the input connection (6) is connectable to the supply connection of the cleaning device (50) and the output connection (8) is connectable to the lumen of the instrument to be cleaned, wherein at least one monitoring sensor (70a) is provided, wherein, in order to monitor at least one fluid parameter of a liquid medium in the interior (9) of the connecting device (1, 1'), interaction of the liquid medium with the monitoring sensor (70a) of the cleaning device (50) can be established via a device (30, 30') of the connecting device (1, 1') when the connecting device (1, 1') is connected in the ready-for-operation state to the at least one supply connection (62a) of the cleaning device (50), **characterized in that** the monitoring sensor (70a) is fixedly arranged in the cleaning chamber (51), and **in that** the interaction of the liquid medium with the monitoring sensor (70a) can be established via a non-permanent connection, the device (30) comprising a plunger (32) mounted in the interior (9) so as to be displaceable against a restoring force, which can assume different displacement positions on the basis of the at least one fluid parameter, in which it projects with an actuating pin (33) to different extents from the connecting device (1, 1') in order to enable interaction with the external monitoring sensor (70a), or the device (30') comprises a nozzle opening (36) through which the liquid medium present in the interior (9) can emerge from the interior (9) as a directed jet (37) in order to enable interaction with the external monitoring sensor (70a).

2. Cleaning device (50) according to claim 1, **characterized in that** the at least one fluid parameter is a pressure.

3. Cleaning device (50) according to claim 1 or 2, **characterized in that** the device (30, 30') of the connecting device (1, 1') is designed to allow the interaction only when the monitored fluid parameter exceeds and/or falls below a minimum value.

4. Cleaning device (50) according to claim 1, 2 or 3, **characterized in that** the interaction is a mechanical operative connection.

5. Cleaning device (50) according to claim 4, **characterized in that** the mechanical interaction is a compressive force.

6. Cleaning device (50) according to claim 4, **characterized in that** there are several output connections (8) for connecting several lumens of one or more instruments to be cleaned.

7. Cleaning device (50) according to one of claims 1 to 6, **characterised in that** the connecting device (1, 1') comprises at least one elongated tube section (2) in which the interior space (9) is formed and at one longitudinal end of which the device (30, 30') is arranged.

8. Cleaning device (50) according to claim 7, **characterised in that** the connecting device (1, 1') comprises a further elongated pipe section (4), which with one of its longitudinal ends is connected substantially at right angles to the at least one pipe section (2), wherein the inlet connection (6) is formed at the free end (5) of the further pipe section (4).

9. Cleaning device (50) according to one of claims 1 to 8, **characterised in that** the supply connection (62a) of the cleaning device (50) is arranged on a rear wall (52) of the cleaning chamber (51) and the monitoring sensor (70a) is arranged on a side wall (54) of the cleaning chamber (51).

10. Cleaning device (50) according to one of claims 1 to 9, **characterized in that** the monitoring sensor (70a) comprises a pressure switch which can be actuated by a pressure force.

11. Cleaning device (50) according to one of the claims 1 to 10, **characterized in that** in the cleaning space (51) there is at least one receptacle (63a) for the arrangement of a cleaning rack (20) with a receiving area (23) for an instrument with a lumen to be cleaned, the cleaning rack (20) comprising a connecting device (1, 1'), and **in that**, in an arrangement of the cleaning basket (20) in the receptacle (63a) ready for operation, the connecting device (1, 1') is connected to the supply connection (62a) ready for operation and is arranged with the device at a measuring range of the monitoring sensor (70a).

12. Cleaning device (50) according to claim 11, **characterized in that** the connecting device (1, 1') is firmly connected to the cleaning rack (20).

13. Cleaning device (50) according to claim 11 or 12, **characterised in that** the connecting device (1, 1') with the device (30, 30') and the input connection (6) is arranged in each case in an edge region of the receiving region (23) of the cleaning rack (20), so that the device (30, 30') and, if applicable, the input connection (6) are accessible from outside the receiving region (23).

14. Cleaning device (50) according to one of claims 11 to 13, which is designed in the manner of a drawer with a largely rectangular base area (21) and has a border with several wall sections (22.1-22.4), so that the cleaning rack (20) can be pushed in and out in a correspondingly designed receptacle (63a) of an insert carriage (60) or of the cleaning chamber (51) in the manner of a drawer.

15. Cleaning device (50) according to claim 14, **characterised in that** the wall sections (22.1-22.4) and/or the base surface (21) comprise a comparatively fine-mesh grid.

16. Cleaning device (50) according to claim 14 or 15, **characterised in that** the connecting device (1, 1') is arranged with the inlet connection (6) at a rear wall section (22.1) and with the device (30, 30') at a lateral wall section (22.3), wherein an opening (28) is formed in the lateral wall section (22.3) at the device (30, 30') and an opening (27) is formed in the rear wall section (22.1) at the inlet connection (6).

17. Cleaning device (50) according to one of claims 11 to 16, **characterised in that** a signalling means (20.1) is provided for signalling a presence of the cleaning rack (20) for detection by a corresponding presence detector of the cleaning device (50).

18. Cleaning device (50) according to claim 17, **characterized in that** the signal means (20.1) is a passive magnetic signal means (20.1).

19. Cleaning device (50) according to one of claims 11 to 18, **characterized in that** further receptacles (63b, 63c) are present, to which a monitoring sensor (70b, 70c) is assigned in each case analogously to the first receptacle (63a), and **in that** each receptacle (63a, 63b, 63c) is provided with a monitoring sensor (70b, 70c), 63c) a connection (62a, 62b, 62c) for providing a liquid medium is assigned to which the respectively associated connecting device (1, 1') is connected in an operable arrangement of the cleaning basket (20) in the respective receptacle (63a, 63b, 63c).

20. Cleaning device (50) according to one of the claims 11 to 19, **characterized in that** the cleaning device (50) comprises an insert carriage (60) for arrangement in the cleaning space (51), on which the at least one receptacle (63a) and the associated connection (62a) are formed, the insert carriage (60) having a distributor connection which can be connected to the supply connection of the cleaning device (50) and is connected in a fluid-conducting manner to the associated connection (62a) via a fluid channel in the insert carriage (60).

21. Cleaning device (50) according to claim 20, **characterized in that** the insert carriage (60) has a carrier (61) on which the distributor connection, at least one receptacle (63a) and the associated connection (62a) are formed and which, in the operational state, can be arranged in the cleaning chamber (51) in such a way that the distributor connection of the insert carriage (60) is connected to the supply connection of the cleaning device (50).

22. Method for cleaning instruments with at least one lumen to be cleaned, to be carried out on a cleaning device according to one of the preceding claims, wherein a connection device is used in the cleaning space (51), comprising a cleaning step in which at least one fluid parameter of the liquid medium in the interior (9) of the connection device (1, 1') is monitored by a monitoring sensor (70a), **characterised in that** the monitoring sensor (70a) is fixedly arranged in the cleaning space (51) and that the at least one fluid parameter is monitored via a non-permanent connection.

23. Method according to claim 22, **characterized in that** exceeding and/or falling below a predetermined minimum value of the fluid parameter is monitored.

24. A method according to claim 22 or 23, **characterized in that** the fluid parameter is a pressure.

## Revendications

1. Dispositif de nettoyage (50) avec une chambre de nettoyage (51) pour le nettoyage d'instruments avec au moins une lumière à nettoyer, dans lequel est prévu au moins un raccord d'alimentation par lequel un milieu liquide peut être mis à disposition et qui est conçu pour le raccordement d'un dispositif de raccordement (1, 1'), dans lequel le dispositif de raccordement (1, 1') présente un espace intérieur (9) avec un raccord d'entrée (6) et au moins un raccord de sortie (8), dans lequel la connexion d'entrée (6) peut être reliée à la connexion d'alimentation du dispositif de nettoyage (50) et la connexion de sortie (8) peut être reliée à la lumière de l'instrument à nettoyer, dans lequel au moins un capteur de surveillance (70a) est prévu, dans lequel, pour surveiller au moins un paramètre fluide d'un milieu liquide à l'intérieur (9) du dispositif de raccordement (1, 1'), une interaction du milieu liquide avec le capteur de surveillance (70a) du dispositif de nettoyage (50) peut être établie par un dispositif (30, 30') du dispositif de raccordement (1, 1') dans lequel le dispositif de raccordement (1, 1') est relié, à l'état prêt à fonctionner, à au moins un raccord d'alimentation (62a) du dispositif de nettoyage (50), **caractérisé en ce que** le capteur de surveillance (70a) est disposé de manière fixe dans la chambre de nettoyage (51), et **en ce que** l'interaction du milieu liquide avec le capteur de surveillance (70a) peut être établie par l'intermédiaire d'une liaison non permanente, le dispositif (30) comprenant un piston (32) monté à l'intérieur (9) de manière à pouvoir être déplacé contre une force de rappel, qui peut prendre différentes positions de déplacement sur la base d'au moins un paramètre du fluide, dans lequel il dépasse du dispositif de raccordement (1, 1') avec un axe d'actionnement (33) à des degrés différents pour permettre une interaction avec le capteur de surveillance externe (70a), ou le dispositif (30') comprend une ouverture de buse (36) à travers laquelle le milieu liquide présent dans l'intérieur (9) peut sortir de l'intérieur (9) sous forme de jet dirigé (37) pour permettre une interaction avec le capteur de surveillance externe (70a).

2. Dispositif de nettoyage (50) selon la revendication 1, **caractérisé en ce que** le au moins un paramètre du fluide est une pression.

3. Dispositif de nettoyage (50) selon la revendication 1 ou 2, **caractérisé en ce que** le moyen (30, 30') du dispositif de raccordement (1, 1') est adapté pour permettre l'interaction uniquement lorsque le paramètre du fluide surveillé dépasse et/ou tombe en dessous d'une valeur minimale.

4. Dispositif de nettoyage (50) selon la revendication 1, 2 ou 3, **caractérisé en ce que** l'interaction est une liaison mécanique fonctionnelle.

5. Dispositif de nettoyage (50) selon la revendication 4, **caractérisé en ce que** l'interaction mécanique est une force de compression.

6. Dispositif de nettoyage (50) selon la revendication 4, **caractérisé en ce qu'**il y a plusieurs connexions de sortie (8) pour connecter plusieurs lumens d'un ou plusieurs instruments à nettoyer.

7. Dispositif de nettoyage (50) selon l'une des revendications 1 à 6, **caractérisé en ce que** le dispositif de raccordement (1, 1') comprend au moins une section de tube allongée (2) dans laquelle l'espace intérieur (9) est formé et à une extrémité longitudinale de laquelle le dispositif (30, 30') est disposé.

8. Dispositif de nettoyage (50) selon la revendication 7, **caractérisé en ce que** le dispositif de raccordement (1, 1') comprend une autre section de tube allongée (4), qui est reliée par l'une de ses extrémités longitudinales sensiblement à angle droit à la au moins une section de tube (2), le raccord d'entrée (6) étant formé à l'extrémité libre (5) de l'autre section de tube (4).

9. Dispositif de nettoyage (50) selon l'une des revendications 1 à 8, **caractérisé en ce que** le raccord d'alimentation (62a) du dispositif de nettoyage (50) est disposé sur une paroi arrière (52) de la chambre de nettoyage (51) et le capteur de surveillance (70a) est disposé sur une paroi latérale (54) de la chambre de nettoyage (51).

10. Dispositif de nettoyage (50) selon l'une des revendications 1 à 9, **caractérisé en ce que** le capteur de surveillance (70a) comprend un interrupteur à pression qui peut être actionné par une force de pression.

11. Dispositif de nettoyage (50) selon l'une des revendications 1 à 10, **caractérisé en ce que** dans l'espace de nettoyage (51) se trouve au moins un réceptacle (63a) pour l'agencement d'un panier de nettoyage (20) avec une zone de réception (23) pour un instrument avec une lumière à nettoyer, le panier de nettoyage (20) comprenant un dispositif de raccordement (1, 1'), et **en ce que**, dans une disposition du panier de nettoyage (20) dans le réceptacle (63a) prêt à fonctionner, le dispositif de raccordement (1, 1') est relié au raccord d'alimentation (62a) prêt à fonctionner et est disposé avec le dispositif sur une plage de mesure du capteur de surveillance (70a).

12. Dispositif de nettoyage (50) selon la revendication 11, **caractérisé en ce que** le dispositif de raccordement (1, 1') est fermement relié au panier de nettoyage (20).

13. Dispositif de nettoyage (50) selon la revendication 11 ou 12, **caractérisé en ce que** le dispositif de liaison (1, 1') avec le dispositif (30, 30') et le raccord d'entrée (6) est disposé dans une zone de bord de la zone de réception (23) du panier de nettoyage (20), de sorte que le dispositif (30, 30') et, le cas échéant, le raccord d'entrée (6) sont accessibles de l'extérieur de la zone de réception (23).

14. Dispositif de nettoyage (50) selon l'une des revendications 11 à 13, qui est conçu à la manière d'un tiroir avec une surface de base (21) largement rectangulaire et qui présente une bordure avec plusieurs sections de paroi (22.1-22.4), de sorte que le panier de nettoyage (20) peut être introduit et retiré à la manière d'un tiroir dans un réceptacle (63a) conçu de manière correspondante d'un chariot d'insertion (60) ou de la chambre de nettoyage (51).

15. Dispositif de nettoyage (50) selon la revendication 14, **caractérisé en ce que** les sections de paroi (22.1-22.4) et/ou la surface de base (21) comprennent une grille à mailles relativement fines.

16. Dispositif de nettoyage (50) selon la revendication 14 ou 15, **caractérisé en ce que** le dispositif de raccordement (1, 1') est disposé avec le raccord d'entrée (6) sur une section de paroi arrière (22.1) et avec le dispositif (30, 30') sur une section de paroi latérale (22.3), une ouverture (28) étant formée dans la section de paroi latérale (22.3) sur le dispositif (30, 30') et une ouverture (27) étant formée dans la section de paroi arrière (22.1) sur le raccord d'entrée (6).

17. Dispositif de nettoyage (50) selon l'une des revendications 11 à 16, **caractérisé en ce qu'**un moyen de signalisation (20.1) est prévu pour signaler une présence du panier de nettoyage (20) pour la détection par un détecteur de présence correspondant du dispositif de nettoyage (50).

18. Dispositif de nettoyage (50) selon la revendication 17, **caractérisé en ce que** le moyen de signalisation (20.1) est un moyen de signalisation magnétique passif (20.1).

19. Dispositif de nettoyage (50) selon l'une des revendications 11 à 18, **caractérisé en ce que** d'autres récipients (63b, 63c) sont présents, auxquels est associé un capteur de surveillance (70b, 70c), à chaque fois de manière analogue au premier récipient (63a), et **en ce que** chaque récipient (63a, 63b, 63c) est pourvu d'un capteur de surveillance (70b, 70c), 63c) un raccord (62a, 62b, 62c) pour la mise à disposition d'un milieu liquide est attribué, auquel est raccordé le dispositif de raccordement (1, 1') respectivement associé dans une disposition fonctionnelle du panier de nettoyage (20) dans le réceptacle respectif (63a, 63b, 63c) .

20. Dispositif de nettoyage (50) selon l'une des revendications 11 à 19, **caractérisé en ce que** le dispositif de nettoyage (50) comprend un chariot à insérer (60) destiné à être disposé dans l'espace de nettoyage (51), sur lequel sont formés le au moins un logement (63a) et le raccord associé (62a), le chariot à insérer (60) présentant un raccord de distribution qui peut être raccordé au raccord d'alimentation du dispositif de nettoyage (50) et qui est relié de manière conductrice de fluide au raccord associé (62a) par l'intermédiaire d'un canal de fluide dans le chariot à insérer (60).

21. Dispositif de nettoyage (50) selon la revendication 20, **caractérisé en ce que** le chariot d'insertion (60) présente un support (61) sur lequel sont formés le raccord de distribution, au moins un réceptacle (63a) et le raccord associé (62a) et qui, à l'état de fonctionnement, peut être disposé dans la chambre de nettoyage (51) de telle sorte que le raccord de distribution du chariot d'insertion (60) soit relié au raccord d'alimentation du dispositif de nettoyage (50).

22. Procédé pour le nettoyage d'instruments avec au moins une lumière à nettoyer, à effectuer sur un dispositif de nettoyage selon l'une des revendications précédentes, dans lequel un dispositif de raccordement est utilisé dans l'espace de nettoyage (51), comprenant une étape de nettoyage dans laquelle au moins un paramètre fluide du milieu liquide à l'intérieur (9) du dispositif de raccordement (1, 1') est surveillé par un capteur de surveillance (70a), **caractérisé en ce que** le capteur de surveillance (70a) est disposé de manière fixe dans l'espace de nettoyage (51) et **en ce que** le au moins un paramètre fluide est surveillé par le biais d'une liaison non permanente.

23. Procédé selon la revendication 22, **caractérisé en ce que** le dépassement et/ou la chute en dessous d'une valeur minimale prédéterminée du paramètre du fluide est surveillé.

24. Procédé selon la revendication 22 ou 23, **caractérisé en ce que** le paramètre du fluide est une pression.
